# EUROPEAN PATENT APPLICATION

(11) **EP 4 039 679 A1**
(43) Date of publication of application: **10.08.2022**
(21) Application number: 20872187.8
(22) Date of filing: 29.09.2020
(51) Int. Cl.: C07D 401/12, C07D 417/12, C07D 209/14, A61K 31/4439, A61K 31/427, A61K 31/4045, A61K 31/4709, A61P 37/00, A61P 35/00

(54) **NOVEL COMPOUND AND USE THEREOF IN TREATING AUTOIMMUNE DISEASES**

(30) Priority: 04.10.2019 US 201962910537 P
(71) Applicant: Parenchyma Biotech Inc., Busan 47392 (KR)
(72) Inventor: SEO, Su Kil, Busan 47509 (KR); JANG, Won Hee, Busan 48050 (KR); LEE, Soung Min, Busan 47171 (KR); YOON, Eun Hye, Busan 47388 (KR); PARK, Ha Young, Busan 47110 (KR); KIM, Chae Eun, Busan 47379 (KR)
(74) Representative: dompatent von Kreisler Selting Werner - Partnerschaft von Patent- und Rechtsanwälten mbB
(86) International application number: PCT/KR2020/013418
(87) International publication number: WO 2021/066573

(57) **Abstract**

The present invention relates to a novel compound and use thereof for treatment of autoimmue diseases. A pharmaceutical composition for treatment or prevention of autoimmune diseases, which includes the novel compound of the present invention, is expected to not only control inflammation but also recover immune balance as well as damaged tissues, thereby restoring bowel tissue homeostasis.

## Description

### [Technical Field]

The present invention relates to a novel compound and use thereof for treatment of autoimmune diseases.

### [Background Art]

The human body can be protected from pathogens through immune response. Biological defense mechanisms against foreign microorganisms such as viruses and bacteria are normally divided into innate immunity and specific immunity, which are mediated by cytokines mostly secreted from immune-related cells.

The immune system serves to protect the body from antigens, that is, harmful foreign substances. Types of these antigens include bacteria, viruses, toxins, cancer cells, and blood and tissues from other humans or animals. The immune system produces antibodies to destroy these harmful substances. If there are autoimmune disorders, the immune system cannot distinguish between its body organs and harmful antigens, and may destroy normal tissues. Diseases derived through such a response as described above refer to an autoimmune disease.

Aryl hydrocarbon receptor (AHR) is a ligand-dependent transcription factor belonging to PER-ARNT-SIM (PAS) superfamily, and is mainly expressed in immune cells, epithelial cells, endothelial cells, and stromal cells of barrier tissues. AHR is an environmental sensor and detects not only xenobiotic ligands such as environmental pollutants (e.g., dioxins), but also physiological ligands generated from cells, microorganisms, and food.

The inactivated form of AHR forms a complex with Hsp90:XAP2:p23:Src chaperone (AHR chaperone complex) in the cytoplasm, and maintains a structure with high affinity for ligand. When AHR is activated after ligand binding, the complex moves to the nucleus and the AHR is isolated from a chaperone complex and binds to AHR-responsive DNA elements (xenobiotic response elements, XREs) located in the upstream regulatory regions of a target gene to regulate the expression of the target gene. Non-toxic immunomodulatory ligands that can activate AHR in vivo may be developed as a new therapeutic agent for autoimmune diseases.

### [Summary of Invention]

### [Problems to be Solved by Invention]

An object of the present invention is to provide a novel compound, a stereoisomer or a pharmaceutically acceptable salt thereof.

In addition, another object of the present invention is to provide a novel compound useful for prevention and treatment of autoimmune diseases, a stereoisomer or a pharmaceutically acceptable salt thereof.

Further, another object of the present invention is to provide a pharmaceutical composition for prevention or treatment of autoimmune diseases, including a novel compound, a stereoisomer or a pharmaceutically acceptable salt thereof.

### [Means for Solving Problems]

1. A compound represented by Formula 1 below, a stereoisomer or a pharmaceutically acceptable salt thereof: 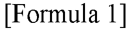 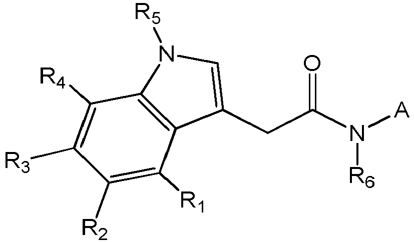 (wherein R₁ to R₄ are each independently hydrogen or halogen, R₅ and R₆ are each independently hydrogen or C₁ - C₅ alkyl,
   A is a single or double cyclic group of C₅ - C₁₂,
   each ring of the cyclic group is substituted with 1 to 3 heteroatoms, and
   the cyclic group is substituted with halogen, C₁-C₅ alkyl or C₁-C₅ alkoxy).
2. The compound, the stereoisomer or the pharmaceutically acceptable salt thereof according to the above 1, wherein A is selected from the group consisting of the following cyclic groups: 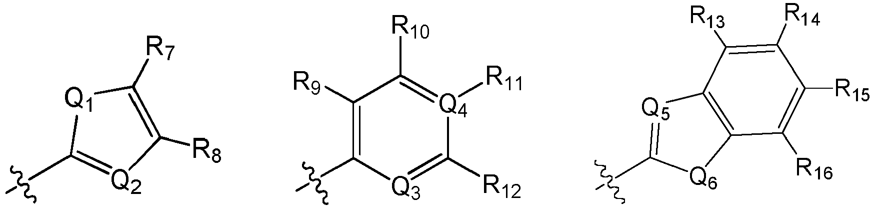 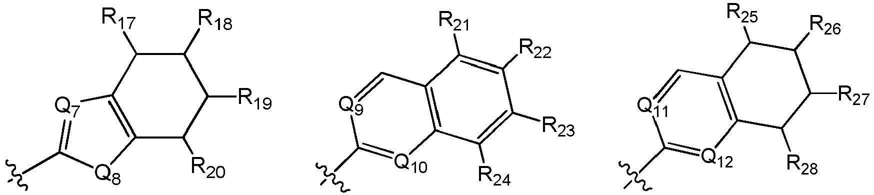 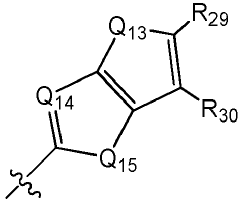 (wherein Q₁ to Q₁₅ are each independently C, N or S, and R₇ to R₃₀ are each independently hydrogen, halogen, C₁-C₃ alkyl or C₁-C₃ alkoxy, and if Q₄ is N, R₁₁ is absent).
3. The compound, the stereoisomer or the pharmaceutically acceptable salt thereof according to the above 1, wherein A is selected from the group consisting of the following cyclic groups: 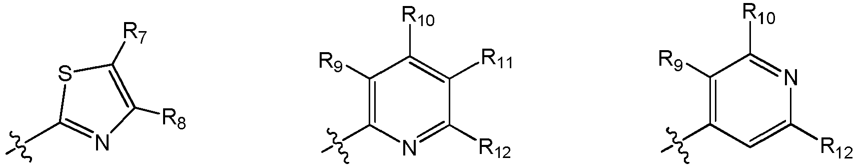 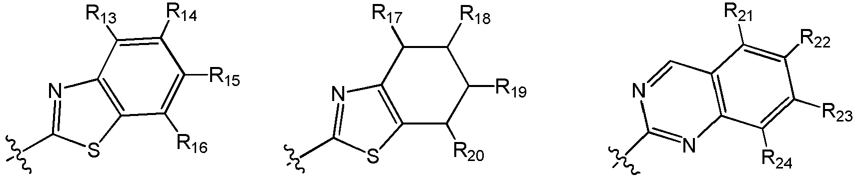 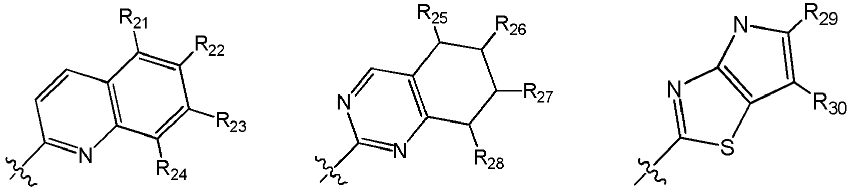 (wherein R₇ to R₃₀ are each independently hydrogen, halogen, C₁-C₃ alkyl or C₁-C₃ alkoxy).
4. The compound, the stereoisomer or the pharmaceutically acceptable salt thereof according to the above 1, wherein A is selected from the group consisting of the following cyclic groups: 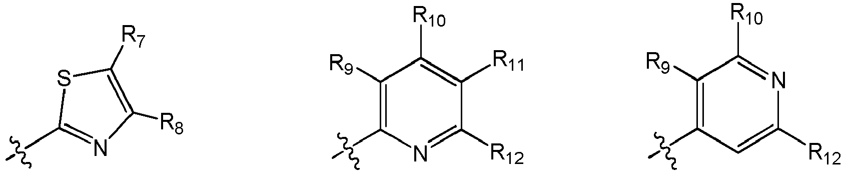 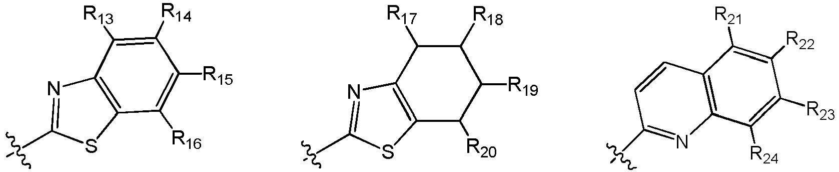 (wherein R₇ to R₂₄ are each independently hydrogen, halogen, C₁-C₃ alkyl or C₁-C₃ alkoxy).
5. The compound, the stereoisomer or the pharmaceutically acceptable salt thereof according to the above 1, wherein A is selected from the group consisting of the following cyclic groups: 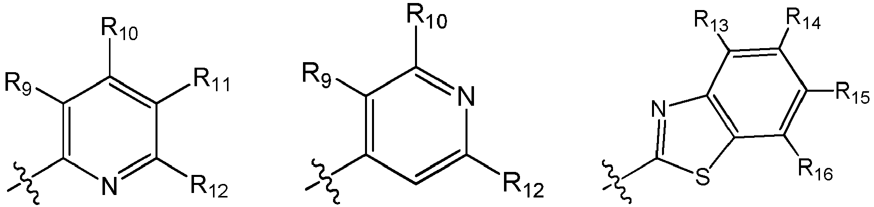 (wherein R₉ to R₁₆ are each independently hydrogen, halogen, C₁-C₃ alkyl or C₁-C₃ alkoxy).
6. The compound, the stereoisomer or the pharmaceutically acceptable salt thereof according to the above 1, wherein R₂ and R₃ are each independently F, Cl or Br.
7. The compound, the stereoisomer or the pharmaceutically acceptable salt thereof according to the above 1, wherein the compound is selected from the group consisting of the following compounds:
   N-(5-bromo-6-methylpyridin-2-yl)-2-(1-methyl-1H-indol-3-yl)acetamide,
   N-(5-bromo-6-methylpyridin-2-yl)-2-(1H-indol-3-yl)acetamide;
   N-(5-bromo-6-methylpyridin-2-yl)-2-(5-chloro-1H-indol-3-yl)acetamide;
   N-(benzo[di]thiazol-2-yl)-2-(1H-indol-3-yl)acetamide;
   N-(benzo[di]thiazol-2-yl)-2-(5-chloro-1H-indol-3-yl)acetamide;
   N-(5-chloro-6-fluoropyridin-2-yl)-2-(1H-indol-3-yl)acetamide;
   2-(5-chloro-1H-indol-3-yl)-N-(5-chloro-6-fluoropyridin-2-yl)acetamide;
   2-(1H-indol-3-yl)-N-(3,4,5-trimethoxyphenyl)acetamide;
   2-(5-chloro-1H-indol-3-yl)-N-(3,4,5-trimethoxyphenyl)acetamide;
   N-(3,5-dichlorophenyl)-2-(1H-indol-3-yl)acetamide;
   2-(5-chloro-1H-indol-3-yl)-N-(3,5-dichlorophenyl)acetamide;
   N-(5-bromo-6-methylpyridin-2-yl)-2-(5-fluoro-1H-indol-3-yl)acetamide;
   2-(5-chloro-1H-indol-3-yl)-N-(pyridin-4-yl)acetamide;
   N-(benzo[di]thiazol-2-yl)-N-methyl-2-(1-methyl-1H-indol-3-yl)acetamide;
   N-(benzo[di]thiazol-2-yl)-2-(1H-indol-3-yl)-N-methyl acetamide;
   N-(benzo[di]thiazol-2-yl)-2-(5-chloro-1H-indol-3-yl)-N-methyl acetamide;
   N-(5-chloro-6-fluoropyridin-2-yl)-2-(1H-indol-3-yl)-N-methyl acetamide;
   2-(5-chloro-1H-indol-3-yl)-N-(5-chloro-6-fluoropyridin-2-yl)-N-methyl acetamide;
   N-(benzo[di]thiazol-2-yl)-2-(5-chloro-1-methyl-1H-indol-3-yl)-N-methyl acetamide;
   N-(5-chloro-6-fluoropyridin-2-yl)-N-methyl-2-(1-methyl-1H-indol-3-yl)acetamide;
   2-(5-chloro-1-methyl-1H-indol-3-yl)-N-(5-chloro-6-fluoropyridin-2-yl)-N-methyl acetamide;
   2-(5-chloro-1-methyl-1H-indol-3-yl)-N-(5-chloro-6-fluoropyridin-2-yl)acetamide;
   N-(benzo[di]thiazol-2-yl)-2-(1-methyl-1H-indol-3-yl)acetamide;
   N-(benzo[di]thiazol-2-yl)-2-(5-fluoro-1H-indol-3-yl)-N-methyl acetamide;
   N-(benzo[di]thiazol-2-yl)-2-(5-chloro-1-methyl-1H-indol-3-yl)acetamide;
   N-(benzo[di]thiazol-2-yl)-2-(5-fluoro-1H-indol-3-yl)acetamide;
   N-(benzo[di]thiazol-2-yl)-2-(6-chloro-1H-indol-3-yl)acetamide;
   2-(5-chloro-1H-indol-3-yl)-N-(thiazol-2-yl)acetamide;
   2-(5-chloro-1H-indol-3-yl)-N-(quinolin-2-yl)acetamide; and
   2-(5-chloro-1H-indol-3-yl)-N-(4,5,6,7-tetrahydrobenzo[di]thiazol-2-yl)acetamide.
8. A pharmaceutical composition, including the compound, the stereoisomer or the pharmaceutically acceptable salt thereof according to any one of the above 1 to 7.
9. The pharmaceutical composition according to the above 8, wherein the composition is used for treating or preventing autoimmune diseases.
10. The pharmaceutical composition according to the above 8, wherein the autoimmune disease is any one selected from the group consisting of multiple sclerosis, inflammatory bowel disease, graft-versus-host disease, asthma, atopy, psoriasis, rheumatoid arthritis, systemic lupus erythematous and type 1 diabetes.
11. The pharmaceutical composition according to the above 8, wherein the composition is used for treating or preventing cancer.
12. The pharmaceutical composition according to the above 11, wherein the cancer is selected from the group consisting of melanoma, colon cancer, liver cancer, gliocytoma, ovarian cancer, colon cancer, head and neck cancer, bladder cancer, kidney cell cancer, stomach cancer, breast cancer, metastatic cancer, prostate cancer, gallbladder cancer, pancreatic cancer, blood cancer, skin cancer and lung cancer.
13. A method for treatment of autoimmune diseases, including administering the compound, the stereoisomer or the pharmaceutically acceptable salt thereof according to any one of the above 1 to 7 to a subject in need thereof.
14. The method for treatment of autoimmune diseases according to the above 13, wherein the autoimmune diseases are selected from the group consisting of multiple sclerosis, inflammatory bowel disease, graft-versus-host disease, asthma, atopy, psoriasis, rheumatoid arthritis, systemic lupus erythematous and type 1 diabetes.
15. A method for inducing AHR, including administering the compound, the stereoisomer or the pharmaceutically acceptable salt thereof according to any one of the above 1to 7 to a subject in need thereof.
16. A method for inhibiting production of IL-6, including administering the compound, the stereoisomer or the pharmaceutically acceptable salt thereof according to any one of the above 1 to 7 to a subject in need thereof.
17. A method for treatment of a cancer, including administering the compound, the stereoisomer or the pharmaceutically acceptable salt thereof according to any one of the above 1 to 7 to a subject in need thereof.
18. The method for treatment of a cancer according to the above 17, wherein the cancer is selected from the group consisting of melanoma, colon cancer, liver cancer, gliocytoma, ovarian cancer, colon cancer, head and neck cancer, bladder cancer, kidney cell cancer, stomach cancer, breast cancer, metastatic cancer, prostate cancer, gallbladder cancer, pancreatic cancer, blood cancer, skin cancer and lung cancer.

### [Advantageous Effects]

The novel compound, the stereoisomer or the pharmaceutically acceptable salt thereof according to the present invention may induce activity of AHR as an immunomodulatory transcription factor, thereby attaining effects of not only controlling inflammation but also restoring immune balance and damaged tissues.

The novel compound, the stereoisomer or the pharmaceutically acceptable salt thereof according to the present invention may inhibit production of IL-6 as an inflammatory factor, thereby attaining effects of regulating excessive immune response, in particular, autoimmune response.

The novel compound, the stereoisomer or the pharmaceutically acceptable salt thereof according to the present invention may exhibit effects of inducing activity of a regulatory T cell (Treg).

Further, the novel compound, the stereoisomer or the pharmaceutically acceptable salt thereof according to the present invention may exhibit effects of preventing and treating autoimmune diseases by regulating the above inflammatory factors.

### [Brief Description of Drawings]

FIGS. 1 and 2 illustrate measurement of CYP1A1 expression level in order to confirm AHR ligand under cell culture conditions of a compound of the present invention.
FIGS. 3 and 4 illustrate measurement of inflammatory factor IL-6 production inhibitory effects of the compound of the present invention.
FIG. 5 illustrates measurement of Foxp3+ regulatory T cell production effects of the compound of the present invention.
FIGS. 6 and 8 illustrates inflammatory bowel disease treatment effects of the compound of the present invention in an animal model with dextran sodium sulfate (DSS)-induced inflammatory bowel disease. Specifically, FIG. 6 demonstrates that the lower the severity index, the more the treatment is completed, as compared to the control (vehicle), while FIG. 7 shows that the smaller the weight of the colon as compared to the length thereof, the higher the treatment effects of inflammatory bowel disease are.
FIGS. 8 and 9 illustrate effects of the compounds of the present invention on inhibiting the expression of inflammatory factors (IL-1β, IL-6, IL-17a, and TNF-α) and increasing the expression of immunomodulatory factors (EL-10, and Foxp3) in an animal model with DSS-induced inflammatory bowel disease.
FIG. 10 illustrates mucosal healing effects of the compound of the present invention using FITC-dextran in an animal model with DSS-induced inflammatory bowel disease. Herein, it means that the lower the detection degree, the higher the mucosa healing effects are.
FIG. 11 illustrates effects of the compound of the present invention on preventing inflammation-induced colon cancer in an AOM/DSS-colorectal cancer animal model. Herein, it means that the smaller the number of tumors per colon, the more effective it is to prevent colon cancer.
FIG. 12 illustrates effects of the compound of the present invention on treatment of multiple sclerosis in an experimental autoimmune encephalomyelitis (EAE) animal model. Specifically, in order to confirm treatment effects of multiple sclerosis, the severity index is shown as a graph by period. Herein, it means that the lower the severity index the more the treatment is completed, as compared to the control.
FIGS. 13 and 14 illustrate effects of the compounds of the present invention on inhibiting the expression of inflammatory factors (IFN-γ, IL-17a, and IL-1β) and increasing the expression of immunomodulatory factors (IL-10, and Foxp3) in the EAE animal model of FIG. 12.
FIG. 15 is a graph illustrating the severity index measured to confirm therapeutic effects of a graft-versus-host disease (GVHD) in an animal model with the lung-graft-versus-host disease.
FIG. 16 illustrates measurement of the expression levels of IL-6, IL-17a and IL-10 factors by the compound of the present invention in the animal model of FIG. 15.

### [Mode for Carrying out Invention]

Hereinafter, the present invention will be described in detail.

All technical terms used in the present invention are used in the same meaning as those skilled in the art may generally understand in the related field of the present invention unless otherwise defined. Further, although preferred methods or samples will be described in the present specification, those similar or equivalent are also included in the scope of the present invention.

The present invention relates to a compound represented by Formula 1 below, a stereoisomer or a pharmaceutically acceptable salt thereof:

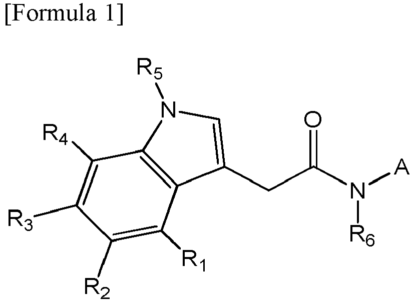

In the above formula, if any substituent is not indicated in a site although the site needs a substituent, it means that a hydrogen substituent is omitted, which would be applied to all formulae in the present invention.

In the above formula, R₁ to R₄ may be each independently hydrogen or halogen, and specifically, hydrogen, fluorine, or chlorine, but they are not limited thereto.

In the above formula, R₅ and R₆ may be each independently hydrogen or C₁-C₅ alkyl, specifically, hydrogen, methyl or ethyl, and more specifically, hydrogen or methyl, but they are not limited thereto.

In the above formula, A may be a single or double cyclic group of C₅-C₁₂, and specifically, cyclopenta-1,3-diene, benzene, cyclohexane, indene, 4,5,6,7-tetrahydroindene, naphthalene, 1,2,3,4-tetrahydronaphthalene, 1,6-dihydropentalene, etc., but it is not limited thereto.

Each ring of the cyclic group may be substituted with 1 to 3 heteroatoms, and for example, 1 to 3 heteroatoms may be each independently substituted with N, S, O, etc., but they are not limited thereto. The heteroatom means an atom rather than carbon or hydrogen.

Further, a site at which the heteroatom can be substituted may include, specifically, Q₁ to Q₁₅ in the following listed structures, but it is not limited thereto.

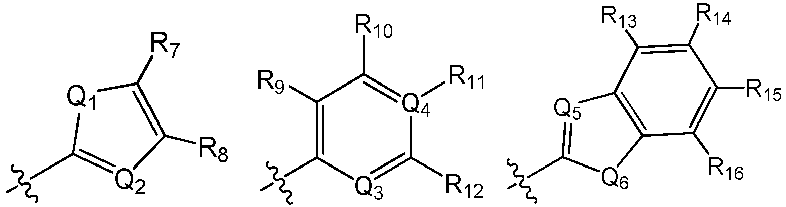

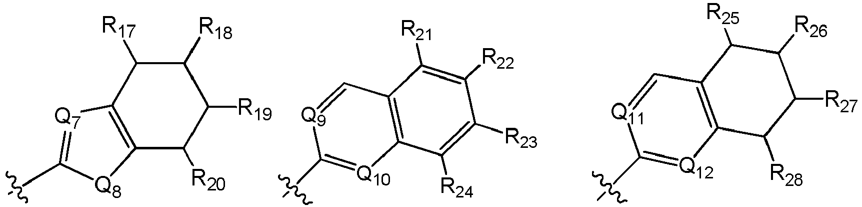

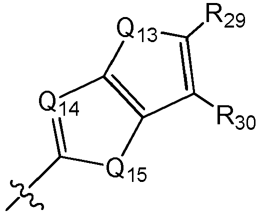

In the above formula, if Q₄ is N, no further substitution could be present at the Q₄ site, thus this may be a case where R₁₁ does not exist.

The cyclic group may be substituted with C₁-C₅ alkyl or C₁-C₅ alkoxy, for example, F, Cl, methyl, ehyl, methoxy, etc., but it is not limited thereto.

A site of the cyclic group which can be substituted with halogen, C₁-C₅ alkyl or C₁-C₅ alkoxy, may specifically include R₇ to R₃₀, but it is not limited thereto.

According to an embodiment of the present invention, A may be selected from the following cyclic groups. 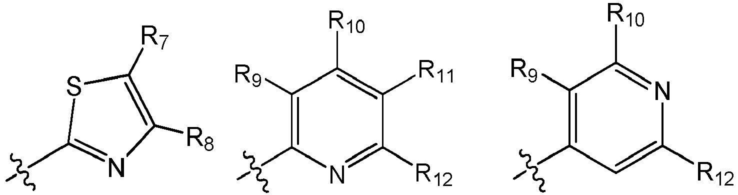 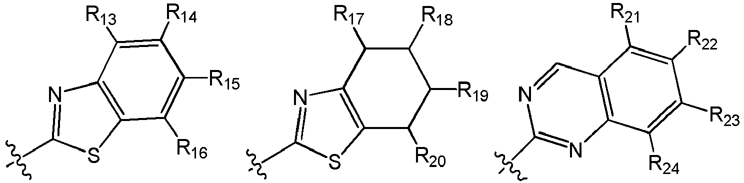 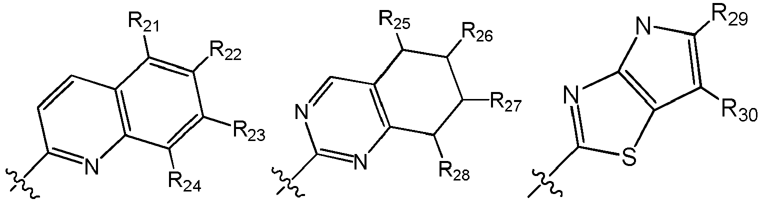 (wherein R₇ to R₃₀ are each independently hydrogen, halogen, C₁-C₃ alkyl or C₁-C₃ alkoxy).

According to an embodiment of the present invention, A may be specifically selected from the following cyclic groups. 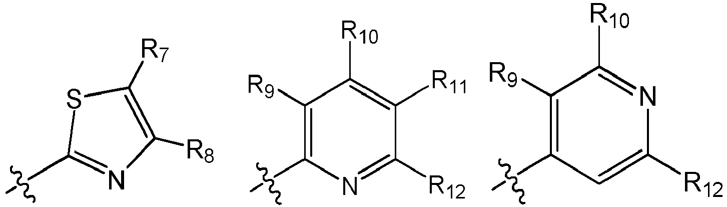 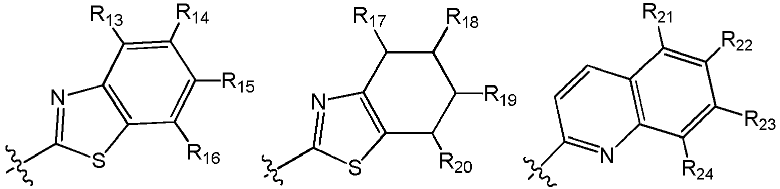 (wherein R₇ to R₂₄ are each independently hydrogen, halogen, C₁-C₃ alkyl or C₁-C₃ alkoxy).

Further, according to an embodiment of the present invention, A may be more specifically selected from the following cyclic groups. 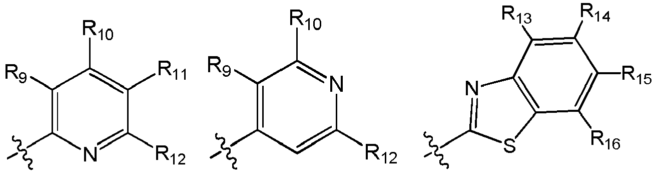 (wherein R₉ to R₁₆ are each independently hydrogen, halogen, C₁-C₃ alkyl or C₁-C₃ alkoxy).

Table 1 below exhibits examples of the structures of compounds represented by Formula 1, which are specifically defined through a combination of R₁ to R₆ and A.

**[TABLE 1]**

| **No.** | **A** | **R₁** | **R₂** | **R3** | **R₄** | **R₅** | **R₆** |
|---|---|---|---|---|---|---|---|
| 1 | 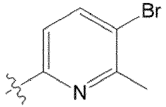 | F | H | H | H | H | H |
| 2 | | H | F | H | H | H | H |
| 3 | | H | H | Br | H | H | H |
| 4 | | H | H | H | F | H | H |
| 5 | | H | H | H | H | H | H |
| 6 | | H | H | H | H | CH₃ | H |
| 7 | | Cl | H | H | H | H | H |
| 8 | | H | Cl | H | H | H | H |
| 9 | | H | H | Cl | Br | H | H |
| 10 | | H | H | H | Cl | H | H |
| 11 | | H | Cl | H | Br | H | H |
| 12 | | H | H | H | H | CH₃ | H |
| 13 | | Cl | H | H | H | H | CH₃ |
| 14 | | H | Cl | H | H | H | CH₃ |
| 15 | | H | Br | H | H | H | CH₃ |
| 16 | | H | H | H | Cl | H | CH₃ |
| 17 | | H | H | H | H | H | CH₃ |
| 18 | | H | H | H | H | CH₃ | CH₃ |
| 19 | 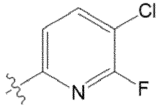 | F | H | H | H | H | H |
| 20 | | H | F | H | H | H | H |
| 21 | | H | Br | H | H | H | H |
| 22 | | H | H | H | F | H | H |
| 23 | | H | H | H | H | H | H |
| 24 | | H | H | H | H | CH₃ | H |
| 25 | | Cl | H | H | H | H | H |
| 26 | | H | Cl | H | H | H | H |
| 27 | | H | Cl | H | F | H | H |
| 28 | | H | H | H | Cl | H | H |
| 29 | | H | H | H | H | H | H |
| 30 | | H | H | H | H | CH₃ | H |
| 31 | | Cl | H | H | H | H | CH₃ |
| 32 | | H | Cl | H | H | H | CH₃ |
| 33 | | H | Br | H | H | H | CH₃ |
| 34 | | H | H | H | Cl | H | CH₃ |
| 35 | | H | H | H | H | H | CH₃ |
| 36 | | H | H | H | H | CH₃ | CH₃ |
| 37 | | H | Cl | H | H | CH₃ | CH₃ |
| 38 | | H | Cl | H | H | CH₃ | H |
| 39 | 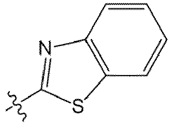 | Cl | H | H | H | H | H |
| 40 | | H | Cl | H | H | H | H |
| 41 | | H | H | Cl | H | H | H |
| 42 | | H | H | H | Cl | H | H |
| 43 | | H | H | H | H | H | H |
| 44 | | H | H | H | H | H | CH₃ |
| 45 | | H | H | H | H | CH₃ | CH₃ |
| 46 | | Cl | H | H | H | H | CH₃ |
| 47 | | H | Cl | H | H | H | CH₃ |
| 48 | | H | H | Cl | H | H | CH₃ |
| 49 | | H | H | H | Cl | H | CH₃ |
| 50 | | Cl | H | H | H | CH₃ | CH₃ |
| 51 | | H | Cl | H | H | CH₃ | CH₃ |
| 52 | | H | H | Cl | H | CH₃ | CH₃ |
| 53 | | H | H | H | Cl | CH₃ | CH₃ |
| 54 | | H | H | H | H | CH₃ | H |
| 55 | | Cl | H | H | H | CH₃ | H |
| 56 | | H | Cl | H | H | CH₃ | H |
| 57 | | H | H | Cl | H | CH₃ | H |
| 58 | | H | H | H | Cl | CH₃ | H |
| 59 | | F | H | H | H | H | CH₃ |
| 60 | | H | F | H | H | H | CH₃ |
| 61 | | H | H | F | H | H | CH₃ |
| 62 | | H | H | H | F | H | CH₃ |
| 63 | | F | H | H | H | H | H |
| 64 | | H | F | H | H | H | H |
| 65 | | H | H | F | H | H | H |
| 66 | | H | H | H | F | H | H |
| 67 | 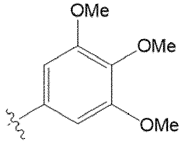 | Cl | H | H | H | H | H |
| 68 | | H | Cl | H | H | H | H |
| 69 | | H | H | Cl | H | H | H |
| 70 | | H | H | H | Cl | H | H |
| 71 | | H | H | H | H | H | H |
| 72 | | Cl | H | H | H | H | CH₃ |
| 73 | | H | Cl | H | H | H | CH₃ |
| 74 | | H | H | Cl | H | H | CH₃ |
| 75 | | H | H | H | Cl | H | CH₃ |
| 76 | | H | H | H | H | H | CH₃ |
| 77 | 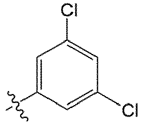 | Cl | H | H | H | H | H |
| 78 | | H | Cl | H | H | H | H |
| 79 | | H | H | Cl | H | H | H |
| 80 | | H | H | H | Cl | H | H |
| 81 | | H | H | H | H | H | H |
| 82 | | Cl | H | H | H | H | CH₃ |
| 83 | | H | Cl | H | H | H | CH₃ |
| 84 | | H | H | Cl | H | H | CH₃ |
| 85 | | H | H | H | Cl | H | CH₃ |
| 86 | | H | H | H | H | H | CH₃ |
| 87 | 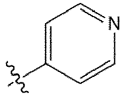 | Cl | H | H | H | H | H |
| 88 | | H | Cl | H | H | H | H |
| 89 | | H | H | Cl | H | H | H |
| 90 | | H | H | H | Cl | H | H |
| 91 | | H | H | H | H | H | H |
| 92 | | Cl | H | H | H | H | CH₃ |
| 93 | | H | Cl | H | H | H | CH₃ |
| 94 | | H | H | Cl | H | H | CH₃ |
| 95 | | H | H | H | Cl | H | CH₃ |
| 96 | | H | H | H | H | H | CH₃ |
| 97 | | H | H | H | H | CH₃ | CH₃ |
| 98 | 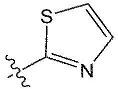 | Cl | H | H | H | H | H |
| 99 | | H | Cl | H | H | H | H |
| 100 | | H | H | Cl | H | H | H |
| 101 | | H | H | H | Cl | H | H |
| 102 | | H | H | H | H | H | H |
| 103 | 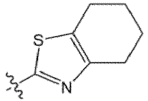 | Cl | H | H | H | H | H |
| 104 | | H | Cl | H | H | H | H |
| 105 | | H | H | Cl | H | H | H |
| 106 | | H | H | H | Cl | H | H |
| 107 | | H | H | H | H | H | H |
| 108 | 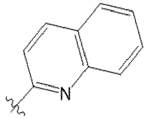 | Cl | H | H | H | H | H |
| 109 | | H | Cl | H | H | H | H |
| 110 | | H | H | Cl | H | H | H |
| 111 | | H | H | H | Cl | H | H |
| 112 | | H | H | H | H | H | H |
| 113 | | H | H | H | H | H | CH₃ |
| 114 | 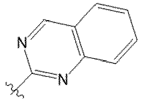 | Cl | H | H | H | H | H |
| 115 | | H | Cl | H | H | H | H |
| 116 | | H | H | Cl | H | H | H |
| 117 | | H | H | H | Cl | H | H |
| 118 | | H | H | H | H | H | H |
| 119 | 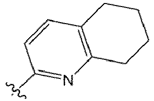 | Cl | H | H | H | H | H |
| 120 | | H | Cl | H | H | H | H |
| 121 | | H | H | Cl | H | H | H |
| 122 | | H | H | H | Cl | H | H |
| 123 | | H | H | H | H | H | H |
| 124 | 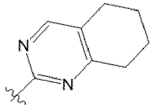 | Cl | H | H | H | H | H |
| 125 | | H | Cl | H | H | H | H |
| 126 | | H | H | Cl | H | H | H |
| 127 | | H | H | H | Cl | H | H |
| 128 | | H | H | H | H | H | H |

The present invention relates to a compound selected from the group consisting of the following compounds, and a stereoisomer or a pharmaceutically acceptable salt thereof.
N-(5-bromo-6-methylpyridin-2-yl)-2-(1-methyl-1H-indol-3-yl)acetamide;
N-(5-bromo-6-methylpyridin-2-yl)-2-(1H-indol-3-yl)acetamide;
N-(5-bromo-6-methylpyridin-2-yl)-2-(5-chloro-1H-indol-3-yl)acetamide;
N-(benzo[di]thiazol-2-yl)-2-(1H-indol-3-yl)acetamide;
N-(benzo[di]thiazol-2-yl)-2-(5-chloro-1H-indol-3-yl)acetamide;
N-(5-chloro-6-fluoropyridin-2-yl)-2-(1H-indol-3-yl)acetamide;
2-(5-chloro-1H-indol-3-yl)-N-(5-chloro-6-fluoropyridin-2-yl)acetamide;
2-(1H-indol-3-yl)-N-(3,4,5-trimethoxyphenyl)acetamide;
2-(5-chloro-1H-indol-3-yl)-N-(3,4,5-trimethoxyphenyl)acetamide;
N-(3,5-dichlorophenyl)-2-(1H-indol-3-yl)acetamide;
2-(5-chloro-1H-indol-3-yl)-N-(3,5-dichlorophenyl)acetamide;
N-(5-bromo-6-methylpyridin-2-yl)-2-(5-fluoro-1H-indol-3-yl)acetamide;
2-(5-chloro-1H-indol-3-yl)-N-(pyridin-4-yl)acetamide;
N-(benzo[di]thiazol-2-yl)-N-methyl-2-(1-methyl-1H-indol-3-yl)acetamide;
N-(benzo[di]thiazol-2-yl)-2-(1H-indol-3-yl)-N-methyl acetamide;
N-(benzo[di]thiazol-2-yl)-2-(5-chloro-1H-indol-3-yl)-N-methyl acetamide;
N-(5-chloro-6-fluoropyridin-2-yl)-2-(1H-indol-3-yl)-N-methyl acetamide;
2-(5-chloro-1H-indol-3-yl)-N-(5-chloro-6-fluoropyridin-2-yl)-N-methyl acetamide;
N-(benzo[di]thiazol-2-yl)-2-(5-chloro-1-methyl-1H-indol-3-yl)-N-methyl acetamide;
N-(5-chloro-6-fluoropyridin-2-yl)-N-methyl-2-(1-methyl-1H-indol-3-yl)acetamide;
2-(5-chloro-1-methyl-1H-indol-3-yl)-N-(5-chloro-6-fluoropyridin-2-yl)-N-methyl acetamide;
2-(5-chloro-1-methyl-1H-indol-3-yl)-N-(5-chloro-6-fluoropyridin-2-yl)acetamide;
N-(benzo[di]thiazol-2-yl)-2-(1-methyl-1H-indol-3-yl)acetamide;
N-(benzo[di]thiazol-2-yl)-2-(5-fluoro-1H-indol-3-yl)-N-methyl acetamide;
N-(benzo[di]thiazol-2-yl)-2-(5-chloro-1-methyl-1H-indol-3-yl)acetamide;
N-(benzo[di]thiazol-2-yl)-2-(5-fluoro-1H-indol-3-yl)acetamide;
N-(benzo[di]thiazol-2-yl)-2-(6-chloro-1H-indol-3-yl)acetamide;
2-(5-chloro-1H-indol-3-yl)-N-(thiazol-2-yl)acetamide;
2-(5-chloro-1H-indol-3-yl)-N-(quinolin-2-yl)acetamide; and
2-(5-chloro-1H-indol-3-yl)-N-(4,5,6,7-tetrahydrobenzo[di]thiazol-2-yl)acetamide;

Further, the present invention relates to a pharmaceutical composition which includes the compound, the stereoisomer or the pharmaceutically acceptable salt thereof.

The pharmaceutical composition may be a pharmaceutical composition for treatment or prevention of autoimmune diseases. Specifically, the disease may be multiple sclerosis (MS), inflammatory bowel disease (IBD), graft-versus-host disease (GVHD), asthma, atopy, psoriasis, rheumatoid arthritis (RA), systemic lupus erythematous (SLE), type 1 diabetes mellitus (T1D), Behcet's disease or Sjogren's syndrome. More specifically, the disease may be multiple sclerosis, inflammatory bowel disease, graft-versus-host disease, asthma, atopy, psoriasis, rheumatoid arthritis, systemic lupus erythematous, type 1 diabetes, but it is not limited thereto.

In the present invention, the "autoimmune disease" may cause damage to cells or tissues by humoral immunity, cellular immunity or both thereof. That is, the autoimmune disease is a disease in which an immune system causes improper reaction to autoantigen thus to induce autoimmune response systemically or specifically in specific organs, etc., which may cause chronic inflammation.

The "multiple sclerosis" refers to an inflammatory disease inducing demyelination and scar formation as a sign and symptom in a broad sense, which is caused by damage and/or consumption of fatty myelin sheaths surrounding axons of the brain and spinal cord. Types of the multiple sclerosis may include recurrent palliative multiple sclerosis (RRMS), secondary progressive multiple sclerosis (SPMS), primary progressive multiple sclerosis (PPMS), and progressive recurrent multiple sclerosis (PRMS), but they are not limited thereto.

The "inflammatory bowel disease" refers to a disease in which abnormal chronic inflammation in the intestine repeats improvement and recurrence, and may correspond to one selected from the group consisting of Chron's disease, ulcerative colitis and intestinal Bechet's disease, but it is not limited thereto.

The "graft-versus-host disease" is a disease in which lymphocytes transfused during hematopoietic stem cell transplantation attack a host with deteriorated immune function to cause symptoms such as fever, rash, and abnormalities of liver function, etc., and may invade the skin, lungs, intestines, liver, or the like, but it is not limited thereto.

The "asthma" refers to a disease in which symptoms such as cough and breathing difficulty occur repeatedly due to inflammation of the bronchi when exposed to a specific causative agent, and may be caused by infection, smoking, allergens, etc., but it is not limited thereto.

The "atopy"' refers to atopic dermatitis, and is a representative allergic disease in which symptoms such as itching and dry skin appear as a chronic recurrent inflammatory skin disease.

The "psoriasis" refers to an inflammatory disease that occurs in the skin or joints due to abnormality in the immune system, and may cause problems such as an occurrence of ugly appearance, increased keratin, or erythematous plaques, and accompanying pain. The psoriasis may include any one or more diseases selected from psoriatic arthritis, guttate psoriasis, pustular psoriasis, red skin psoriasis, scalp psoriasis, nail psoriasis and enthesitis.

The "rheumatoid arthritis" refers to a systemic autoimmune disease characterized by chronic inflammation of the joint site.

The "systemic lupus erythematous" is also called as "lupus," and refers to a systemic disease that invades various organs of the body, such as connective tissue, skin, joints, blood and kidneys, as a chronic inflammatory autoimmune disease. The exact cause is not known, but according to previous studies, it is known that genetic factors are associated with the occurrence of this disease. To help diagnose lupus, the American College of Rheumatology (ACR) has published 11 symptoms, signs, and test findings to help differentiate this disease from other diseases. According to the published study, if four or more among the 11 symptoms occur, it could be diagnosed as lupus.

The "type 1 diabetes" is an immune-mediated disease in which insulin-secreting beta cells are destroyed by an autoimmune reaction. The causes of this disease may include a number of genetic and environmental factors, which are specifically targeted to insulin-secreting beta cells. This disease may be accompanied with progressive inflammatory infiltration of the pancreatic islets by the immune cells.

In the present invention, the pharmaceutical composition may be prepared using a pharmaceutically suitable and physiologically acceptable additive in addition to the active ingredient, which is the compound of the present invention. The composition may be administered to a mammal. As the additive described above, for example, excipients, disintegrants, sweeteners, binders, coating agents, swelling agents, lubricants, glidants or flavoring agents may be used.

Further, the pharmaceutical composition of the present invention may be preferably formulated as a pharmaceutical composition that includes at least one pharmaceutically acceptable carrier in addition to the active ingredient in a pharmaceutically effective amount described above for administration.

The "pharmaceutically effective amount" means an amount sufficient to treat a disease with a reasonable benefit/risk ratio applicable to medical treatment, and an effective dose level may be determined based on a type and severity of patient's disease, drug activity, drug sensitivity, time of administration, route of administration and rate of excretion, duration of treatment, factors including drugs used concurrently, and other factors well known in the medical field. The pharmaceutical composition according to the present invention may be administered as an individual therapeutic agent or administered in combination with other therapeutic agents. Further, the composition may be administered sequentially or simultaneously with a conventional therapeutic agent, and may be administered in single or multiple doses. In consideration of all of the above factors, it is important to administer a minimum amount capable of attaining the maximum effect without side effects, such an amount could be easily determined by those skilled in the art.

Specifically, the effective amount of the pharmaceutical composition according to the present invention may vary depending on an age, sex, condition and/or body weight of the patient, absorption of the active ingredient in the body, inactivation rate and excretion rate, type of disease, and the drug to be used in combination. Typically, 0.001 to 150 mg, preferably 0.01 to 100 mg per 1 kg of body weight may be administered daily or every other day, or may be divided into 1 to 3 times a day. However, the dosage may be increased or decreased depending on the route of administration, severity of obesity, sex, body weight, age, etc., therefore, would not limit the scope of the present invention in any way.

Further, the "pharmaceutically acceptable" refers to a composition that is physiologically acceptable and does not usually cause allergic reactions such as gastrointestinal disorders and dizziness, or similar reactions when administered to humans.

Examples of the carrier, excipient and diluent may include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oils. Further, fillers, anti-aggregating agents, lubricants, wetting agents, flavoring agents, emulsifying agents, and preservatives may additionally be included.

Further, the composition of the present invention may be formulated using any method known in the art in order to provide rapid, sustained or delayed release of the active ingredient after administration thereof to a subject in need of treatment using the pharmaceutical composition of the present invention including humans. The formulation may be powder, granule, tablet, emulsion, syrup, aerosol, soft or hard gelatin capsule, sterile injectable solution, sterile powder.

The present invention may relate to a method for treatment of an autoimmune disease, which includes administering the compound, the stereoisomer or the pharmaceutically acceptable salt thereof to a subject in need thereof.

Further, the present invention may relate to a method for inducing activity of AHR, which includes administering the compound, the stereoisomer or the pharmaceutically acceptable salt thereof.

Specifically, the compounds of the present invention may target the aryl hydrocarbon receptor (AHR), which is an immunomodulatory transcription factor of the present invention, and may serve as an agent to induce AHR activity, thereby controlling inflammation, regulating immune balance, and repairing damaged tissue. Therefore, the compound may be used for therapeutic purposes, but it is not limited thereto. Existing ligands are toxic, have low affinity and structural stability, and high target non-specificity, which entail a problem in that these are unsuitable for development into pharmaceutical compositions. On the other hand, when AHR activity is induced by the compound of the present invention having "drug-like properties," it could be effectively used for treatment and prevention of autoimmune diseases.

The present invention may relate to a method for inhibiting production of IL-6, which includes administering the compound, the stereoisomer or the pharmaceutically acceptable salt thereof.

Specifically, the compound of the present invention is known to cause autoimmune diseases by IL-6, an inflammatory factor, and thus may be used in treatment of autoimmune diseases through a mechanism that inhibits the production thereof. Actually, there is a number of known therapeutic agents for autoimmune diseases that target inhibition of IL-6, as well as related papers. According to the following experimental data, the compound of the present invention is also confirmed to inhibit the production of IL-6 and thus is expected to have effects of reducing the autoimmune response, whereby the composition of the present invention may be used for treatment and prevention of autoimmune diseases.

Further, the present invention relates to a composition for prevention or treatment of a cancer, which includes the compound, the stereoisomer or the pharmaceutically acceptable salt thereof

In the present invention, "cancer" broadly refers to uncontrolled abnormal growth of the host's own cells that invade the surrounding tissues of the initial abnormal cell growth site in the host and potential tissues located distally of these sites. Further, carcinoma as a cancer of epithelial tissues (e.g., the skin, squamous cells); sarcoma, as a cancer of connective tissue (e.g., bone, cartilage, fat, muscle, blood vessels, etc.); leukemia as a cancer of blood-forming tissue (e.g., bone marrow tissue); lymphoma and myeloma, which are cancers of immune cells; cancers of the central nervous system, including cancers in the brain and spinal tissue, may be included.

Specifically, the cancer may be selected from the group consisting of melanoma, colon cancer, liver cancer, gliocytoma, ovarian cancer, colon cancer, head and neck cancer, bladder cancer, kidney cell cancer, stomach cancer, breast cancer, metastatic cancer, prostate cancer, gallbladder cancer, pancreatic cancer, blood cancer, skin cancer and lung cancer, but it is not limited thereto.

The present invention relates to a method for treatment of a cancer, which includes administering the compound, the stereoisomer or the pharmaceutically acceptable salt thereof to a subject in need thereof.

The treatment method may include administering the compound, the stereoisomer or the pharmaceutically acceptable salt thereof to a patient, who was diagnosed with cancer, at any stage of chemotherapy, and it is not limited to a specific stage.

Further, the compound, the stereoisomer or the pharmaceutically acceptable salt thereof may be administered in the aforementioned forms of the pharmaceutical composition, but it is not limited thereto.

The compound represented by Formula 1 according to the present invention may be prepared by any method known in various documents. In the following preparative examples, the synthetic methods for some of the compounds listed in Table 1 have been briefly described, however, they are not limited thereto.

Hereinafter, the present invention will be described in detail by means of preparative examples and examples of the present invention.

### Preparative Example

### 1. Synthesis of N-(5-bromo-6-methylpyridin-2-yl)-2-(5-chloro-1H-indol-3-yl)acetamide (compound 8)

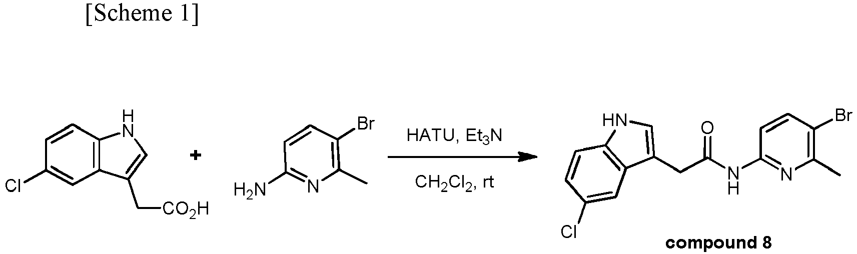

While stirring a solution of 2-(5-chloro-1H-indol-3-yl)acetic acid (1.00 g, 4.77 mmol) in CH₂Cl₂ (30 mL) at room temperature, 5-bromo-6-methylpyridin-2-amine (892 mg, 4.77 mmol), 1-[bis(dimethylamino)methylene]-1H-1,2,3-triazole[4,5-b]pyridinium-3-oxidehexafluorophosphate (HATU, 2.18 g, 5.72 mmol) and trimethylamine (1.33 mL, 9.54 mmol) were sequentially added dropwise. The reaction mixture was stirred at room temperature for 3 days, and distilled water (10 mL) was added to the mixture to terminate the reaction. The layers were separated, and the organic layer was washed with distilled water, dried over anhydrous Na₂SO₄ and filtered. After concentrating the filtrate under reduced pressure, the concentrate was purified by column chromatography (SiO₂, hexanes:EtOAc = 4:1 - 2:1) to yield a light gray compound (970 mg, 54%).

¹H NMR (CDCl₃, 400 MHz): δ 8.67 (br s, 1H), 8.00 (d, J = 8.0 Hz, 1H), 7.97 (br s, 1H), 7.55 (d, J = 4.0 Hz, 1H), 7.23 (d, J = 8.0 Hz, 1H), 7.14 (m, 2H), 3.84 (s, 2H), 2.45 (s, 3H).

### 2. Synthesis of N-(benzo[di]thiazol-2-yl)-2-(5-chloro-1H-indol-3-yl)acetamide (compound 40)

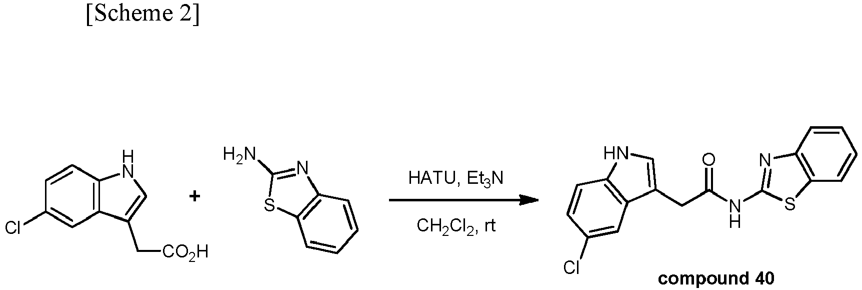

A title compound with white color (1.31 g, 80%) was obtained by the same experimental procedures as in Preparative Example 1, except that the amine of Preparative Example 1 was altered to benzo[di]thiazol-2-amine.

¹H NMR (DMSO-d6, 400 MHz): δ 12.58 (br s, 1H), 11.20 (br s, 1H), 7.95 (m, 1H), 7.74 (d, J = 8.0 Hz, 1H), 7.68 (d, J = 4.0 Hz, 1H), 7.43 (ddd, J = 8.0, 8.0, 2.0 Hz, 1H), 7.39 (m, 2H), 7.29 (ddd, J = 8.0, 8.0, 2.0 Hz, 1H), 7.09 (dd, J = 8.0, 4.0 Hz, 1H), 3.91 (s, 2H).

### 3. Synthesis of 2-(5-chloro-1H-indol-3-yl)-N-(5-chloro-6-fluoropyridin-2-yl)acetamide (compound 26)

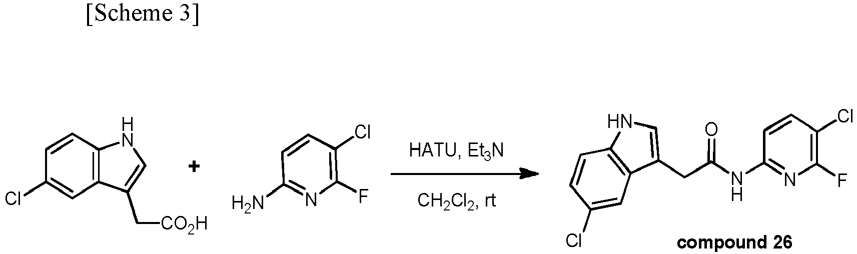

A title compound with light yellow color (560 mg, 35%) was obtained by the same experimental procedures as in Preparative Example 1, except that the amine of Preparative Example 1 was altered to 5-chloro-6-fluoropyridine-2-amine.

¹H NMR (CDCl₃, 400 MHz): δ 8.38 (br s, 1H), 8.14 (dd, J = 8.0, 2.0 Hz, 1H), 7.87 (br s, 1H), 7.77 (dd, J = 8.0, 8.0 Hz, 1H), 7.54 (dd, J = 4.0, 2.0 Hz, 1H), 7.33 (dd, J = 8.0, 0.8 Hz, 1H), 7.21 (m, 2H), 3.87 (s, 2H).

### 4. Synthesis of N-(5-bromo-6-methylpyridin-2-yl)-2-(5-fluoro-1H-indol-3-yl)acetamide (compound 2)

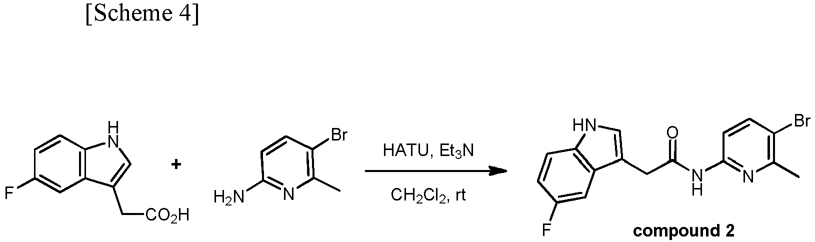

A title compound with light brown color (107 mg, 44%) was obtained by the same experimental procedures as in Preparative Example 1, except that the acetic acid of Preparative Example 1 was altered to 2-(5-chloro-1H-indol-3-yl)acetic acid.

¹H NMR (CDCl₃, 400 MHz): δ 9.09 (br s, 1H), 8.20 (br s, 1H), 8.02 (d, J = 8.0 Hz, 1H), 7.76 (d, J = 8.0 Hz, 1H), 7.20 (dd, J = 8.0, 4.0 Hz, 1H), 7.13 (dd, J = 8.0, 4.0 Hz, 1H), 6.98 (d, J = 4.0 Hz, 1H), 6.89 (m, 1H), 3.83 (s, 2H), 2.44 (s, 3H).

### 5. Synthesis of N-(benzo[di]thiazol-2-yl)-N-methyl-2-(1-methyl-1H-indol-3-yl)acetamide (compound 45)

While stirring a solution of N-(benzo[di]thiazol-2-yl)-2-(1H-indol-3-yl)acetamide (70.0 mg, 0.228 mmol) in DMF (1 mL) at room temperature under an argon atmosphere, t-BuOK (74.0 mg, 0.456 mmol) was added dropwise and stirred for 5 minutes. MeI (28.4 µL, 0.456 mmol) was added dropwise to the mixture and stirred for 30 minutes. Distilled water (1 mL) was added to the mixture to terminate the reaction. The layers were separated, and the organic layer was washed with distilled water, dried over anhydrous MgSO₄ and filtered. After concentrating the filtrate under reduced pressure, the concentrate was purified by column chromatography (SiO₂, hexane:EtOAc = 4:1) to yield the title compound with white color (39.0 mg, 51%).

¹H NMR (CDCl₃, 500 MHz): δ 7.83 (d, J = 8.1 Hz, 1H), 7.80 (d, J = 7.9 Hz, 1H), 7.64 (d, J = 8.0 Hz, 1H), 7.43 (td, J = 7.8, 1.0 Hz, 1H), 7.29 (m, 3H), 7.16 (t, J = 7.5 Hz, 1H), 4.17 (s, 2H), 3.85 (s, 3H), 3.77 (s, 3H).

¹³C NMR (CDCl₃, 125 MHz): δ 171.87, 160.26, 148.19, 137.08, 133.59, 127.73, 127.56, 126.01, 123.92, 122.20, 121.36, 121.18, 119.61, 118.76, 109.60, 105.76, 35.73, 32.93, 32.87.

### 6. Synthesis of N-(5-chloro-6-fluoropyridin-2-yl)-2-(1H-indol-3-yl)-N-methyl acetamide (compound 35)

### (1) Stage 1: Synthesis of 2-(1H-indol-3-yl)acetyl chloride

While stirring a solution of indol-3-acetic acid (39.3 mg, 0.224 mmol) in CH₂Cl₂ (1.5 mL) at 0 °C under an argon atmosphere, oxalyl chloride (96.0 µL, 1.12 mmol) and DMF (1 drop) were sequentially added dropwise. The reaction mixture was stirred for 1 hour. The mixture was concentrated under reduced pressure, dried in vacuum and used in the next reaction without further purification.

### (2) Stage 2: Synthesis of N-(5-chloro-6-fluoropyridin-2-yl)-2-(1H-indol-3-yl)-N-methyl acetamide

While stirring a solution of 5-chloro-6-fluoro-N-methylpyridin-2-amine (30.0 mg, 0.187 mmol) in THF (1 mL) at 0 °C under an argon atmosphere, n-BuLi (116 µL, 0.187 mmol) was added dropwise. The reaction mixture was stirred for 1 hour. The solution of 2-(1H-indol-3-yl)acetyl chloride in CH₂Cl₂ (0.5 mL) in stage 1 was added dropwise to the mixture. After the mixture was stirred for 5 minutes, distilled water (1 mL) was added to terminate the reaction. The layers were separated, and the organic layer was washed with distilled water, dried over anhydrous MgSO₄, and filtered. After concentrating the filtrate under reduced pressure, the concentrate was purified by column chromatography (SiO₂, hexane:EtOAc:CH₂Cl₂ =3:3:1) to yield a title compound with brown color (19.0 mg, 27%).

¹H NMR (CDCl₃, 500 MHz): δ 8.15 (s, 1H), 7.69 (t, J = 8.7 Hz, 1H), 7.53 (d, J = 7.9 Hz, 1H), 7.34 (s, 1H), 7.31 (s, 1H), 7.19 (t, J = 7.5 Hz, 1H), 7.11 (t, J = 7.4 Hz, 1H), 7.04 (s, 1H), 3.98 (s, 2H), 3.43 (s, 3H).

¹³C NMR (CDCl₃, 125 MHz): δ 172.17, 159.28, 157.85, 142.29, 141.91, 141.90, 136.20, 127.17, 122.94, 122.44, 119.86, 118.77, 118.01, 117.97, 117.77, 117.73, 111.36, 108.76, 35.60, 29.83.

### 7. Synthesis of 2-(5-chloro-1H-indol-3-yl)-N-(5-chloro-6-fluoropyridin-2-yl)-N-methyl acetamide (compound 32)

A title compound with brown color (11.6 mg, 15%) was obtained by the same experimental procedures as in Preparative Example 6, except that the acetic acid of Preparative Example 6 was altered to 2-(5-chloro-1H-indol-3-yl)acetic acid.

¹H NMR (CDCl₃, 500 MHz): δ 8.25 (s, 1H), 7.82 (t, J = 8.6 Hz, 1H), 7.74 (t, J = 8.7 Hz, 1H), 7.46 (s, 1H), 7.22 (d, J = 8.6 Hz, 1H), 7.12 (dd, J = 8.6, 1.6 Hz, 1H), 7.06 (s, 1H), 3.92 (s, 2H), 3.43 (s, 3H).

¹³C NMR (CDCl₃, 125 MHz): δ 159.06, 157.93, 156.01, 151.70, 151.60, 142.09, 134.54, 128.35, 125.61, 124.52, 122.72, 119.05, 118.34, 117.99, 117.95, 117.26, 112.39, 35.67, 29.82.

### 8. Synthesis of N-(benzo[di]thiazol-2-yl)-2-(5-chloro-1-methyl-1H-indol-3-yl)-N-methyl acetamide (compound 51)

A title compound with white color (26.4 mg, 54%) was obtained by the same experimental procedures as in Preparative Example 5 while using N-(benzo[di]thiazol-2-yl)-2-(5-chloro-1H-indol-3-yl)acetamide.

¹H NMR (CDCl₃, 500 MHz): δ 7.83 (d, J = 8.1 Hz, 1H), 7.80 (d, J = 7.9 Hz, 1H), 7.58 (d, J = 1.0 Hz, 1H), 7.43 (t, J = 7.6 Hz, 1H), 7.30 (t, J = 7.5 Hz, 1H), 7.23 (d, J = 8.7 Hz, 1H), 7.20 (dd, J = 8.7, 1.5 Hz, 1H), 7.08 (s, 1H), 4.11 (s, 2H), 3.87 (s, 3H), 3.75 (s, 3H).

¹³C NMR (CDCl₃, 125 MHz): δ 171.51, 160.22, 148.12, 135.51, 133.56, 129.19, 128.65, 126.09, 125.58, 124.03, 122.55, 121.42, 121.23, 118.28, 110.71, 105.57, 35.73, 33.16, 32.49.

### 9. Synthesis of N-(5-chloro-6-fluoropyridin-2-yl)-N-methyl-2-(1-methyl-1H-indol-3-yl)acetamide (compound 36)

A title compound with yellow color (7.2 mg, 54%) was obtained by the same experimental procedures as in Preparative Example 5 while using N-(5-chloro-6-fluoropyridin-2-yl)-2-(1H-indol-3-yl)acetamide.

¹H NMR (CDCl₃, 500 MHz): δ 7.69 (t, J = 8.7 Hz, 1H), 7.50 (d, J = 7.9 Hz, 1H), 7.35 (d, J = 7.0 Hz, 1H), 7.29 (d, J = 8.2 Hz, 1H), 7.23 (t, J = 7.6 Hz, 1H), 7.11 (t, J = 7.4 Hz, 1H), 6.95 (s, 1H), 3.96 (s, 2H), 3.75 (s, 3H), 3.43 (s, 3H).

¹³C NMR (CDCl₃, 125 MHz): δ 172.27, 157.85, 155.94, 151.78, 151.69, 141.87, 141.86, 136.99, 127.65, 127.61, 122.00, 119.36, 118.82, 118.03, 117.98, 112.96, 112.72, 109.46, 107.06, 35.56, 32.87, 32.82.

### 10. Synthesis of 2-(5-chloro-1-methyl-1H-indol-3-yl)-N-(5-chloro-6-fluoropyridin-2-yl)-N-methyl acetamide (compound 37)

A title compound with yellow color (17.7 mg, 56%) was obtained by the same experimental procedures as in Preparative Example 5 while using 2-(5-chloro-1H-indol-3-yl)-N-(5-chloro-6-fluoropyridin-2-yl)acetamide.

¹H NMR (CDCl₃, 500 MHz): δ 7.74 (t, J = 8.7 Hz, 1H), 7.44 (s, 1H), 7.35 (br s, 1H), 7.19 (d, J = 8.6 Hz, 1H), 7.15 (dd, J = 8.7, 1.4 Hz, 1H), 6.98 (s, 1H), 3.90 (s, 2H), 3.72 (s, 3H), 3.43 (s, 3H).

¹³C NMR (CDCl₃, 125 MHz): δ 171.82, 157.89, 155.98, 151.69, 151.64, 142.02, 141.98, 135.39, 129.12, 128.67, 125.28, 122.27, 118.36, 117.98, 117.94, 113.23, 112.96, 110.54, 106.88, 35.61, 33.06, 32.43.

### 11. Synthesis of 2-(5-chloro-1-methyl-1H-indol-3-yl)-N-(5-chloro-6-fluoropyridin-2-yl)acetamide (compound 38)

A title compound with white color (8.20 mg, 27%) was obtained by the same experimental procedures as in Preparative Example 5 while using 2-(5-chloro-1H-indol-3-yl)-N-(5-chloro-6-fluoropyridin-2-yl)acetamide.

¹H NMR (CDCl₃, 500 MHz): δ 8.14 (d, J = 8.5 Hz, 1H), 7.86 (s, 1H), 7.76 (t, J = 8.8 Hz, 1H), 7.51 (s, 1H), 7.27 (d, J = 8.2 Hz, 1H), 7.22 (dd, J = 8.7, 1.5 Hz, 1H), 7.09 (s, 1H), 3.84 (s, 2H), 3.81 (s, 3H).

¹³C NMR (CDCl₃, 125 MHz): δ 169.98, 157.71, 155.80, 147.54, 147.44, 142.93, 135.85, 129.96, 128.47, 126.07, 123.10, 118.22, 111.79, 111.75, 111.00, 110.94, 110.68, 105.82, 34.50, 33.27

### 12. Synthesis of N-(5-bromo-6-methylpyridin-2-yl)-2-(1-methyl-1H-indol-3-yl)acetamide (compound 6)

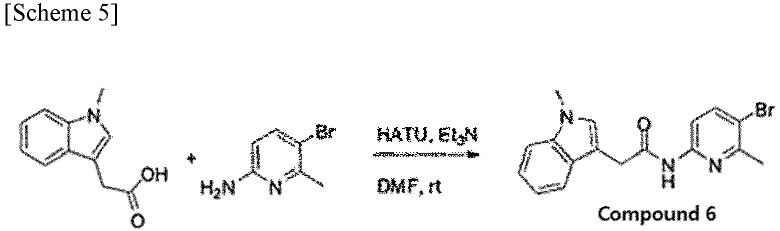

While stirring a solution of 2-(1-methyl-1H-indol-3-yl)acetic acid (200 mg, 1.06 mmol) in DMF (5 mL) at room temperature, 5-bromo-6-methylpyridin-2-amine (197 mg, 1.06 mmol), 1-[bis(dimethylamino)methylene]-1H-1,2,3-triazole[4,5-b]pyridinium-3-oxide hexafluorophosphate (HATU, 402 mg, 1.06 mmol) and trimethylamine (0.3 mL, 2.11 mmol) were sequentially added. The reaction mixture was stirred at room temperature for 3 days. Distilled water was added to the mixture to terminate the reaction. The layers were separated with ethyl acetate, and the organic layer was washed with distilled water, dried over anhydrous Na₂SO₄, and filtered. After concentrating the filtrate under reduced pressure, the concentrate was purified by column chromatography to yield a title compound (120 mg, 31%).

¹H NMR (CDCl₃, 400 MHz): δ 7.99(m,2H), 7.72(d, 1H, J=12.0Hz), 7.58(d, 1H, J=12.0Hz), 7.35(d, 1H, J=8.0Hz), 7.27(m, 1H), 7.15(m,1H), 7.06(s, 1H), 3.87(s, 2H), 3.80(s, 3H), 2.42(s, 3H)

### 13. Synthesis of N-(5-bromo-6-methylpyridin-2-yl)-2-(1H-indol-3-yl)acetamide (compound 5)

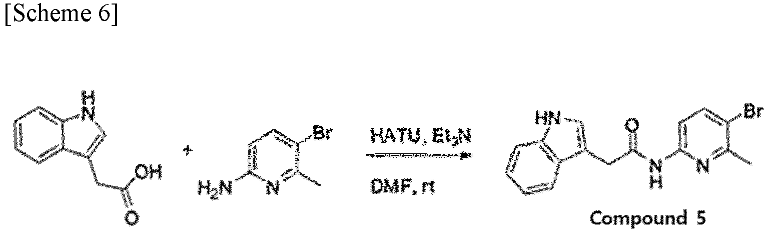

A title compound (110 mg, 30%) was obtained by the same experimental procedures as in Preparative Example 12, except that the acetic acid of Preparative Example 12 was altered to 2-(1H-indol-3-yl)acetic acid.

¹H NMR (CDCl₃, 400 MHz): δ 8.37(s,1H), 7.99(d, 1H, J=12.0Hz), 7.74(d, 1H, J=8.0Hz), 7.60(d, 1H, J=8.0Hz), 7.40(d, 1H, J=8.0Hz), 7.25(m, 1H), 7.16(m,1H), 3.87(s, 2H), 3.90(s, 3H), 2.43(s, 3H)

### 14. Synthesis of N-(benzo[di]thiazol-2-yl)-2-(1H-indol-3-yl)acetamide (compound 43)

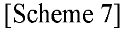

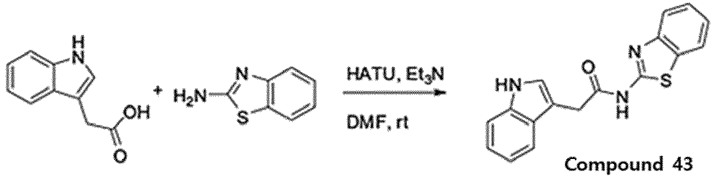

While stirring a solution of 2-(1H-indol-3-yl)acetic acid (100 mg, 0.57 mmol) in DMF (3 mL) at room temperature, benzo[di]thiazol-2-amine (197 mg, 0.57 mmol), 1-[bis(dimethylamino)methylene]-1H-1,2,3-triazole[4,5-b]pyridinium-3-oxide hexafluorophosphate (HATU, 260 mg, 0.68 mmol) and trimethylamine (0.16 mL, 1.14 mmol) were added sequentially. The reaction mixture was stirred at room temperature for 3 days. Distilled water was added to the mixture to terminate the reaction. The layers were separated with ethyl acetate, and the organic layer was washed with distilled water, dried over anhydrous Na₂SO₄, and filtered. After concentrating the filtrate under reduced pressure, the concentrate was purified by column chromatography to yield a title compound (10 mg, 6%).

¹H NMR (CDCl₃, 400 MHz): δ 8.99(s,1H), 8.32(s, 1H) 7.80(d, 1H, J=8.0Hz), 7.64(d, 1H, J=8.0Hz), 7.56(d, 1H, J=12.0Hz), 7.40(m, 2H), 7.29(m, 3H), 7.16(m,1H), 4.03(s, 2H)

### 15. Synthesis of N-(5-chloro-6-fluoropyridin-2-yl)-2-(1H-indol-3-yl)acetamide (compound 23)

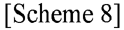

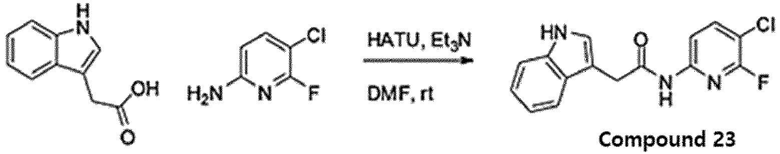

A title compound (6 mg, 3%) was obtained by the same experimental procedures as in Preparative Example 14, except that the amine of Preparative Example 14 was altered to 5-chloro-6-fluoropyridin-2-amine.

¹H NMR (CDCl₃, 400 MHz): δ8.14(dd,1H, J=8.0Hz and 2.0Hz), 7.95(s, 1H) 7.75(m, 1H), 7.57(d, 1H, J=8.0Hz), 7.44(m, 1H), 7.24(m, 2H), 7.16(m, 1H), 3.92(s, 2H)

### 16. Synthesis of 2-(1H-indol-3-yl)-N-(3,4,5-trimethoxyphenyl)acetamide (compound 71)

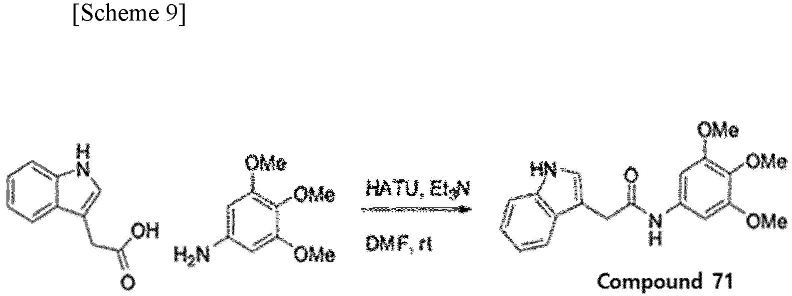

A title compound (10 mg, 5%) was obtained by the same experimental procedures as in Preparative Example 14, except that the amine of Preparative Example 14 was altered to 3,4,5-trimethoxyaniline.

¹H NMR (DMSO-d6, 400 MHz): δ 7.59(d,1H, J=8.0Hz), 7.35(d,1H, J=8.0Hz), 7.25(m, 1H) 7.07(m, 1H), 7.00(s, 1H), 6.97(m, 1H), 3.71(s, 6H), 3.69(s, 2H), 3.59(s, 3H)

### 17. Synthesis of 2-(5-chloro-1H-indol-3-yl)-N-(3,4,5-trimethoxyphenyl)acetamide (compound 68)

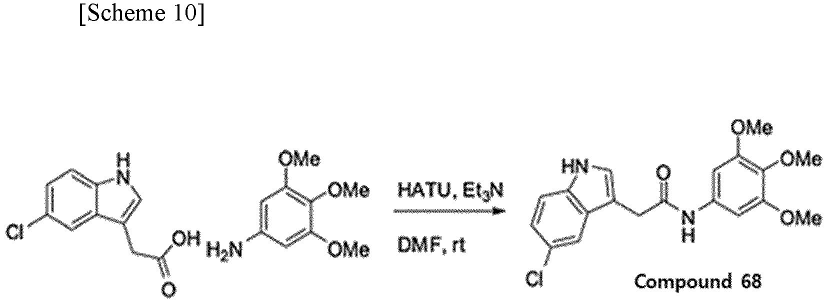

While stirring a solution of 2-(5-chloro-1H-indol-3-yl)acetic acid (100 mg, 0.47 mmol) in DMF (3 mL) at room temperature, 3,4,5-trimethoxyaniline (87 mg, 0.47 mmol), 1-[bis(dimethylamino)methylene]-1H-1,2,3-triazole[4,5-b]pyridinium-3-oxide hexafluorophosphate (HATU, 217 mg, 0.57 mmol) and trimethylamine (0.13 mL, 0.95 mmol) were added sequentially. The reaction mixture was stirred at room temperature for 3 days. Distilled water was added to the mixture to terminate the reaction. The layers were separated with ethyl acetate, and the organic layer was washed with distilled water, dried over anhydrous Na₂SO₄, and filtered. After concentrating the filtrate under reduced pressure, the concentrate was purified by column chromatography to yield a title compound (10 mg, 5%).

¹H NMR (DMSO-d6, 400 MHz): δ 11.12(s, 1H), 10.05(s, 1H), 7.65(d,1H, J=4.0Hz), 7.38(s, 1H), 7.36(s, 1H),7.07(m, 1H), 6.99(s, 2H), 3.72(s, 6H), 3.68(s, 2H), 3.33(s, 3H)

### 18. Synthesis of N-(3,5-dichlorophenyl)-2-(1H-indol-3-yl)acetamide (compound 81)

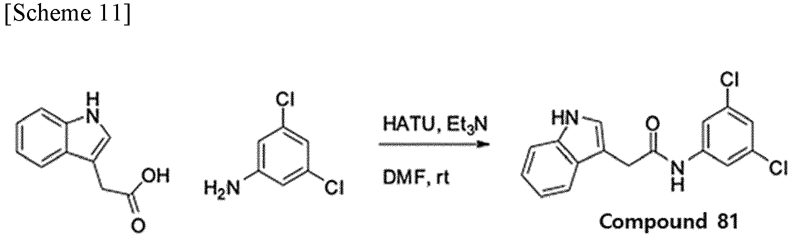

A title compound (8 mg, 5%) was obtained by the same experimental procedures as in Preparative Example 14, except that the amine of Preparative Example 14 was altered to 3,5-dichloroaniline.

¹H NMR (CDCl₃, 400 MHz): δ 8.50(s,1H), 7.60(s, 1H), 7.55(d, 1H, J=8.0Hz), 7.41(d, 1H, J=8.0Hz), 7.30(d, 1H, J=4.0Hz), 7.24(m, 1H), 7.16(m, 2H), 7.01(m,1H), 3.86(s, 2H)

### 19. Synthesis of 2-(5-chloro-1H-indol-3-yl)-N-(3,5-dichlorophenyl)acetamide (compound 78)

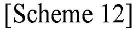

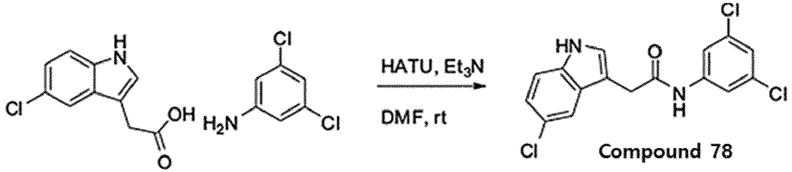

A title compound (10 mg, 6%) was obtained by the same experimental procedures as in Preparative Example 17, except that the amine of Preparative Example 17 was altered to 3,5-dichloroaniline.

¹H NMR (CDCl₃, 400 MHz): δ 8.33(s,1H), 7.56(s, 1H), 7.36(d, 1H, J=8.0Hz), 7.34(d, 1H, J=2.0Hz), 7.30(bs, 1H), 7.26(m, 1H), 7.23(m, 2H), 7.06(m,1H), 3.85(s, 2H)

### 20. Synthesis of 2-(5-chloro-1H-indol-3-yl)-N-(pyridin-4-yl)acetamide (compound 88)

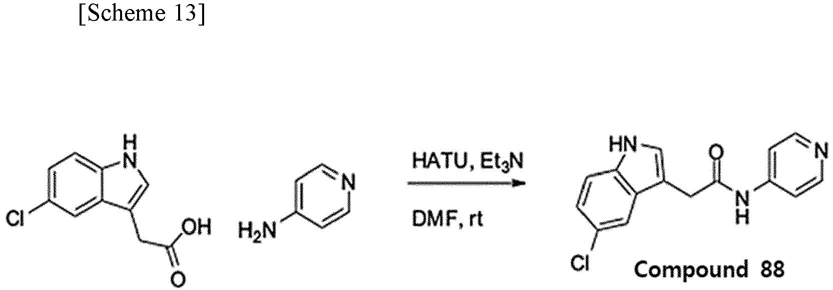

A title compound (10 mg, 7%) was obtained by the same experimental procedures as in Preparative Example 17, except that the amine of Preparative Example 17 was altered to pyridine-4-amine.

¹H NMR (DMSO-d6, 400 MHz): δ 11.15(s, 1H), 10.49(s, 1H), 8.40(m,1H), 7.63(d, 1H, J=2.0Hz), 7.57(m, 1H) 7.37(d, 1H, J=8.0Hz), 7.34(d, 1H, J=4.0Hz),7.07(dd, 1H, J=12.0Hz and 2.0Hz), 3.72(s, 6H), 3.77(s, 2H)

### 21. Synthesis of N-(benzo[di]thiazol-2-yl)-2-(1H-indol-3-yl)-N-methyl acetamide (compound 44)

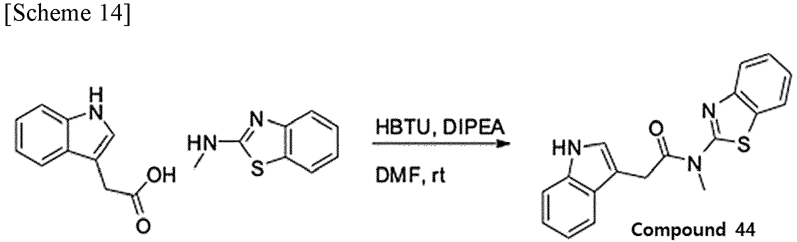

While stirring a solution of 2-(1H-indol-3-yl)acetic acid (960 mg, 5.48 mmol) in DMF (35 mL) at room temperature, N-methylbenzo[di]thiazol-2-amine (600 mg, 3.65 mmol), N,N,N',N'-tetramethyl-O-(1H-benzotrazol-1-yl)euronium hexafluorophosphate (HBTU, 2.77 g, 7.31 mmol) and N,N-diisopropylethylamine (2.55 mL, 14.61 mmol) were added sequentially. The reaction mixture was stirred at room temperature for 3 days. Distilled water was added to the mixture to terminate the reaction. The layers were separated with ethyl acetate, and the organic layer was washed with distilled water, dried over anhydrous Na₂SO₄ and filtered. After concentrating the filtrate under reduced pressure, the concentrate was purified by column chromatography to yield a title compound (460 mg, 39%).

¹H NMR (DMSO-d6, 400 MHz): δ 11.02(s, 1H), 7.93(m,1H), 7.79(m, 1H) 7.57(m, 1H), 7.38(m, 2H), 7.31(m, 2H), 7.08(m, 1H), 6.98(m, 1H), 4.23(s,2H), 3.84(s, 3H)

### 22. Synthesis of N-(benzo[di]thazol-2-yl)-2-(5-chloro-1H-indol-3-yl)-N-metyl acetamide (compound 47)

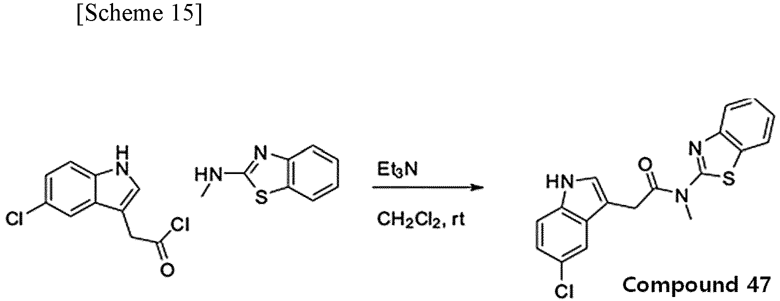

While stirring a solution of N-methylbenzo[di]thiazol-2-amine (100 mg, 0.61 mmol) in CH₂Cl₂ (12 mL) at room temperature, triethylamine (0.65 mL, 3.68 mmol) was added, then 2-(5- chloro-1H-indol-3-yl)acetylchloride (280 mg, 1.23 mmol) was further added dropwise. The reaction mixture was stirred at room temperature for 1 day. Distilled water was added to the mixture to terminate the reaction. The layers were separated, and the organic layer was washed with distilled water, dried over anhydrous Na₂SO₄ and filtered. After concentrating the filtrate under reduced pressure, the concentrate was purified by column chromatography to yield a title compound (30 mg, 7%).

¹H NMR (DMSO-d6, 400 MHz): δ 11.22(s, 1H), 7.95(d,1H, J=8.0Hz), 7.80(d,1H, J=8.0Hz), 7.66(d,1H, J=4.0Hz), 7.42(m, 3H) 7.31(m, 1H), 7.09(dd, 1H, J=8.0Hz and 2.0Hz), 4.25(s,2H), 3.87(s, 3H)

### 23. Synthesis of N-(benzo[di]thiazol-2-yl)-2-(1-methyl-1H-indol-3-yl)acetamide (compound 54)

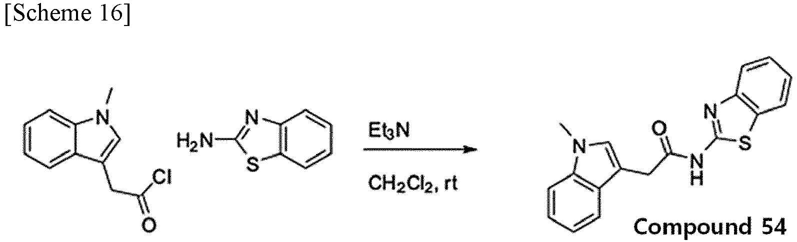

While stirring a solution of benzo[di]thiazol-2-amine (214 mg, 1.43 mmol) in CH₂Cl₂ (15 mL) at room temperature, triethylamine (0.66 mL, 4.75 mmol) was added, then 2-(1-methyl-1H-indol-3-yl)acetylchloride (329 mg, 1.58 mmol) was further added dropwise. The reaction mixture was stirred at room temperature for 1 day. Distilled water was added to the mixture to terminate the reaction. The layers were separated, and the organic layer was washed with distilled water, dried over anhydrous Na₂SO₄ and filtered. After concentrating the filtrate under reduced pressure, the concentrate was purified by column chromatography to yield a title compound (120 mg, 23%).

¹H NMR (CDCl₃, 400 MHz): δ 8.96(s, 1H), 7.80(d,1H, J=8.0Hz), 7.63(d,1H, J=8.0Hz), 7.54(d,1H, J=8.0Hz), 7.30(m, 2H) 7.16(m, 1H), 7.10(s, 1H), 4.01(s,2H), 3.83(s, 3H)

### 24. Synthesis of N-(benzo[di]thiazol-2-yl)-2-(5-fluoro-1H-indol-3-yl)-N-methyl acetamide (compound 60)

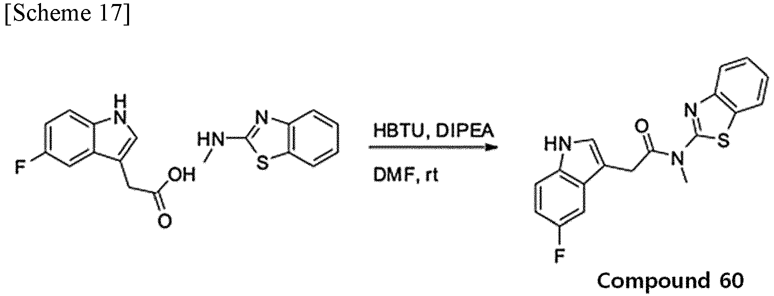

While stirring a solution of 2-(5-fluoro-1H-indol-3-yl)acetic acid (352 mg, 1.83 mmol) in DMF (12 mL) at room temperature, N-methylbenzo[di]thiazol-2-amine (200 mg, 1.22 mmol), N,N,N',N'-tetramethyl-O-(1H-benzotriazol-1-yl)euronium hexafluorophosphate (HBTU, 923 mg, 2.44 mmol) and N,N-diisopropylethylamine (0.9 mL, 4.87 mmol) were sequentially added. The reaction mixture was stirred at room temperature for 3 days. Distilled water was added to the mixture to terminate the reaction. The layers were separated with ethyl acetate, and the organic layer was washed with distilled water, dried over anhydrous Na₂SO₄ and filtered. After concentrating the filtrate under reduced pressure, the concentrate was purified by column chromatography to yield a title compound (140 mg, 33%).

¹H NMR (DMSO-d6, 400 MHz): δ 11.12(s, 1H), 7.95(d,1H, J=8.0Hz), 7.80(d,1H, J=8.0Hz), 7.37(m, 5H) 6.93(m, 1H), 4.23(s,2H), 3.86(s, 3H)

### 25. Synthesis of N-(benzo[di]thiazol-2-yl)-2-(5-chloro-1-methyl-1H-indol-3-yl)acetamide (compound 56)

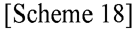

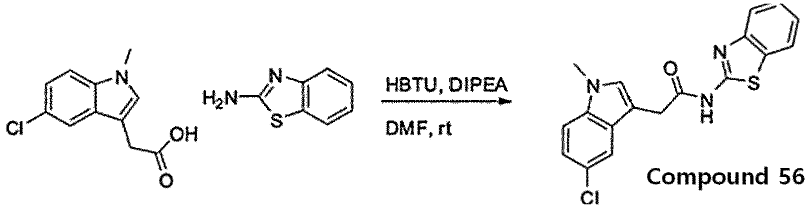

While stirring a solution of 2-(5-chloro-1-methyl-1H-indol-3-yl)acetic acid (300 mg, 1.34 mmol) in DMF (13 mL) at room temperature, benzo[di]thiazol-2-amine (161 mg, 1.07 mmol), N,N,N',N'-tetramethyl-O-(1H-benzotriazol-1-yl)euronium hexafluorophosphate (HBTU, 1.02 g, 2.68 mmol) and N,N-diisopropylethylamine (0.9 mL, 5.37 mmol) were sequentially added. The reaction mixture was stirred at room temperature for 3 days. Distilled water was added to the mixture to terminate the reaction. The layers were separated with ethyl acetate, and the organic layer was washed with distilled water, dried over anhydrous Na₂SO₄ and filtered. After concentrating the filtrate under reduced pressure, the concentrate was purified by column chromatography to yield a title compound (170 mg, 35%).

¹H NMR (CDCl₃, 400 MHz): δ 9.35(s, 1H), 7.81(d,1H, J=8.0Hz), 7.65(d,1H, J=8.0Hz), 7.48(m, 1H), 7.39(m, 1H), 7.25(m, 3H), 7.04(s, 1H), 3.94(s,2H), 3.76(s, 3H)

### 26. Synthesis of N-(benzo[di]thiazol-2-yl)-2-(5-fluoro-1H-indol-3-yl)acetamide (compound 64)

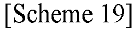

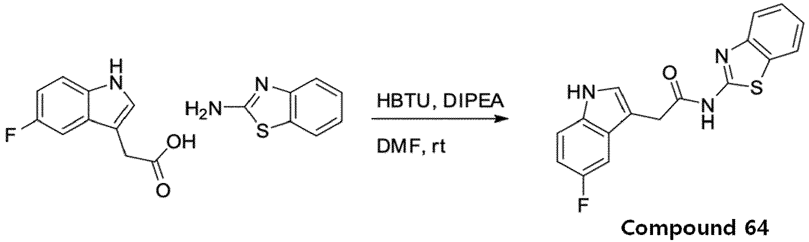

While stirring a solution of 2-(5-fluoro-1H-indol-3-yl)acetic acid (50 mg, 0.25 mmol) in DMF (3 mL) at room temperature, benzo[di]thiazol-2-amine (31 mg, 0.20 mmol), N,N,N',N'-tetramethyl-O-(1H-benzotriazol-1-yl)euronium hexafluorophosphate (HBTU, 196 mg, 0.51 mmol) and N,N- diisopropylethylamine (0.2 mL, 1.04 mmol) were sequentially added. The reaction mixture was stirred at room temperature for 3 days. Distilled water was added to the mixture to terminate the reaction. The layers were separated with ethyl acetate, and the organic layer was washed with distilled water, dried over anhydrous Na₂SO₄ and filtered. After concentrating the filtrate under reduced pressure, the concentrate was purified by column chromatography to yield a title compound (10 mg, 12%).

¹H NMR (DMSO-d6, 400 MHz): δ 12.65(s, 1H), 11.10(s, 1H), 7.95(d,1H, J=8.0Hz), 7.74(d,1H, J=8.0Hz), 7.38(m, 4H), 7.28(m, 1H), 6.93(m, 1H), 3.90(s,2H)

### 27. Synthesis of N-(benzo[di]thiazol-2-yl)-2-(6-chloro-1H-indol-3-yl)acetamide (compound 41)

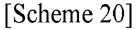

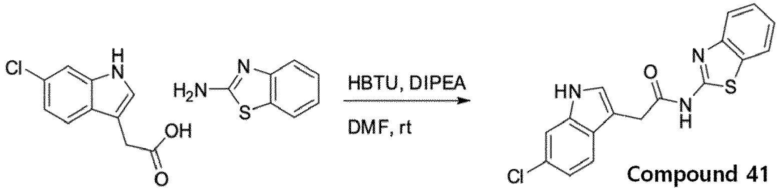

A title compound (6 mg, 3%) was obtained by the same experimental procedures as in Preparative Example 26, except that the acetic acid of Preparative Example 26 was altered to 2-(6-chloro-1H-indol-3-yl)acetic acid.

¹H NMR (MeOD-d4, 400 MHz): δ 11.65(s, 1H), 11.10(s, 1H), 7.85(d,1H, J=8.0Hz), 7.74(d,1H, J=8.0Hz), 7.58(d, 1H, J=8.0Hz), 7.43(m, 2H), 7.31(m, 2H), 7.05(dd, 1H, J=8.0Hz and 4.0Hz), 4.87(s,2H)

### 28. Synthesis of 2-(5-chloro-1H-indol-3-yl)-N-(thiazol-2-yl)acetamide (compound 99)

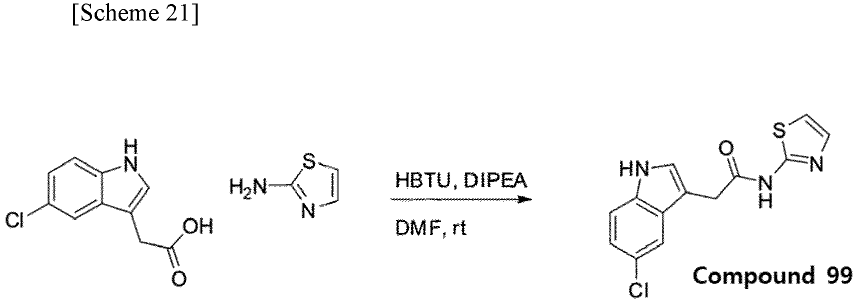

While stirring a solution of 2-(5-chloro-1H-indol-3-yl)acetic acid (125 mg, 0.49 mmol) in DMF (5 mL) at room temperature, thiazol-2-amine (50 mg, 0.59 mmol), N, N,N',N'-tetramethyl-O-(1H-benzotriazol-1-yl)euronium hexafluorophosphate (HBTU, 378 mg, 0.99 mmol) and N,N-diisopropylethylamine (0.4 mL, 2.00 mmol) were added sequentially. The reaction mixture was stirred at room temperature for 3 days. Distilled water was added to the mixture to terminate the reaction. The layers were separated with ethyl acetate, and the organic layer was washed with distilled water, dried over anhydrous Na₂SO₄ and filtered. After concentrating the filtrate under reduced pressure, the concentrate was purified by column chromatography to yield a title compound (19 mg, 13%).

¹H NMR (DMSO-d6, 400 MHz): δ 12.31(s, 1H), 11.18(s, 1H), 7.66(d,1H, J=2.0Hz), 7.46(d,1H, J=4.0Hz), 7.38(s, 1H), 7.36(m, 1H), 7.18(d, 2H, J=4.0Hz), 7.07(dd, 1H, J=8.0Hz and 4.0Hz), 3.84(s,2H)

### 29. Synthesis of 2-(5-chloro-1H-indol-3-yl)-N-(quinolin-2-yl)acetamide (compound 109)

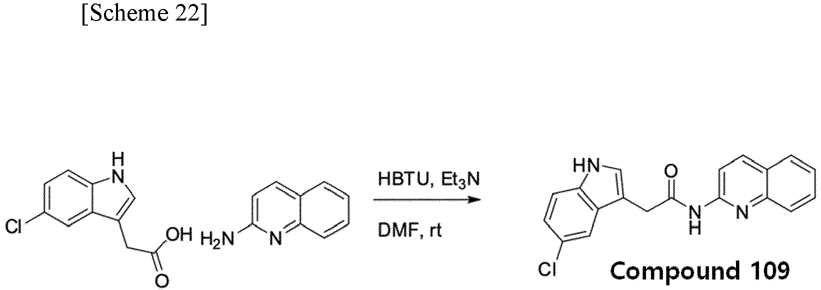

A title compound (18 mg, 15%) was obtained by the same experimental procedures as in Preparative Example 28, except that the amine of Preparative Example 28 was altered to quinoline-2-amine.

¹H NMR (DMSO-d6, 400 MHz): δ 11.16(s, 1H), 10.98(s, 1H), 8.30(m, 1H), 7.89(dd,1H, J=8.0Hz and 2.0Hz), 7.82(d,1H, J=4.0Hz), 7.72(m, 2H), 7.48(m, 1H), 7.40(d, 1H, J=2.0Hz), 7.37(d, 1H, J=12.0Hz),7.07(dd, 1H, J=8.0Hz and 4.0Hz), 3.86(s,2H)

### 30. Synthesis of 2-(5-chloro-1H-indol-3-yl)-N-(4,5,6,7-tetrahydrobenzo[di]thiazol-2-yl)acetamide (compound 104)

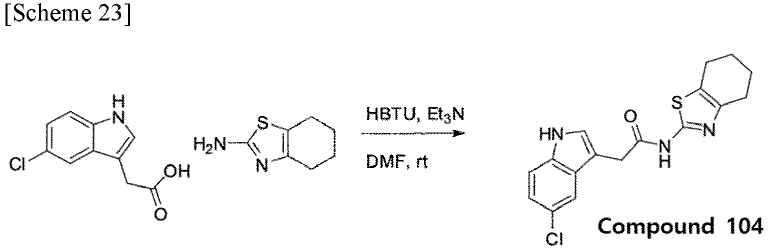

A title compound (19 mg, 17%) was obtained by the same experimental procedures as in Preparative Example 28, except that the amine of Preparative Example 28 was altered to 4,5,6,7-tetrahydrobenzo[di]thiazol-2-amine.

¹H NMR (DMSO-d6, 400 MHz): δ 12.07(s, 1H), 11.17(s, 1H), 7.63(d,1H, J=4.0Hz), 7.37(d, 1H, J=8.0Hz and 2.0Hz), 7.07(dd, 1H, J=8.0Hz and 4.0Hz), 3.78(s,2H), 2.52(m, 8H)

The structures of the above compounds are shown in Tables 2a to 2c, and molecular weights of the compounds are listed in Table 2d.

**[TABLE 2a]**

| **Compound** | **Structure** | **Compound** | **Structure** |
|---|---|---|---|
| 2 | 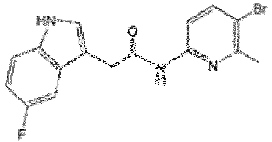 | 35 | 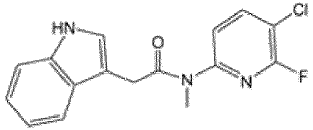 |
| 5 | 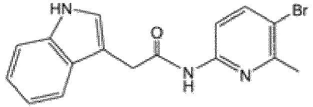 | 36 | 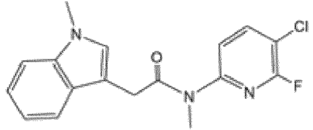 |
| 6 | 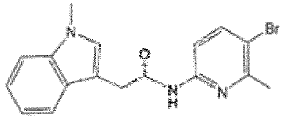 | 37 | 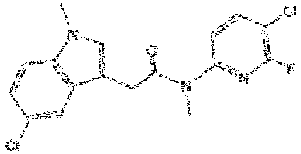 |
| 8 | 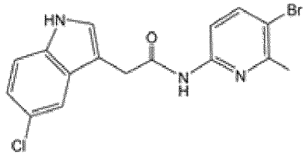 | 38 | 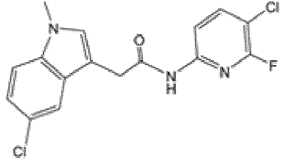 |
| 23 | 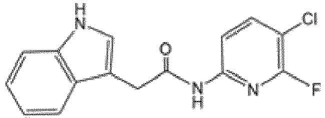 | 40 | 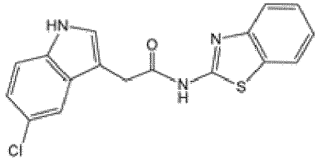 |
| 26 | 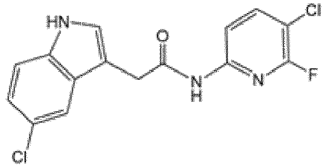 | 41 | 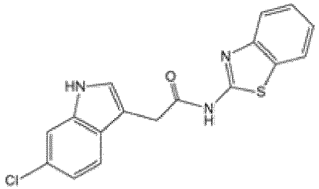 |
| 32 | 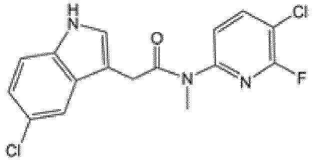 | 43 | 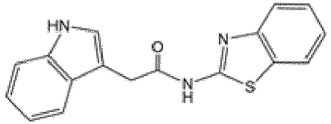 |

**[TABLE 2b]**

| **Compound** | **Structure** | **Compound** | **Structure** |
|---|---|---|---|
| 44 | 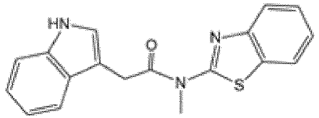 | 64 | 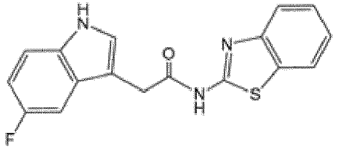 |
| 45 | 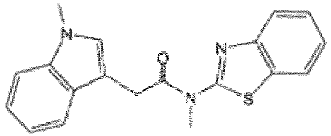 | 68 | 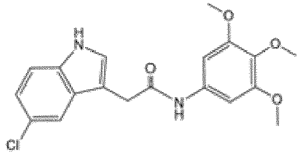 |
| 47 | 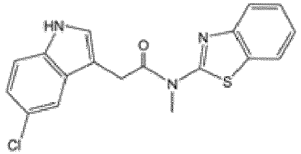 | 71 | 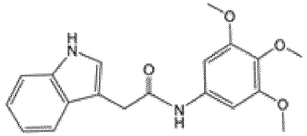 |
| 51 | 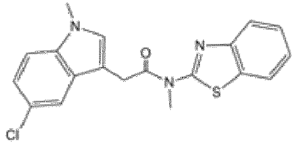 | 78 | 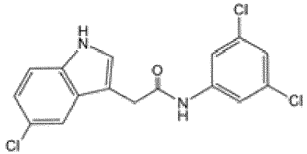 |
| 54 | 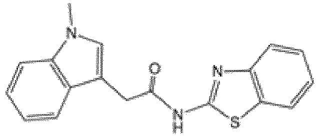 | 81 | 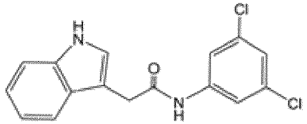 |
| 56 | 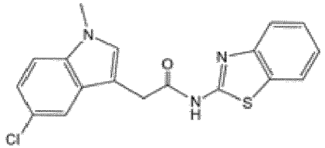 | 88 | 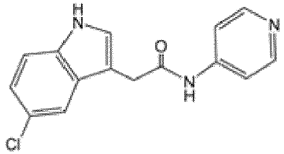 |
| 60 | 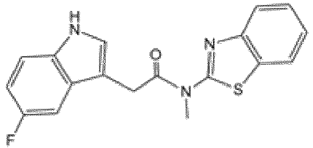 | 99 | 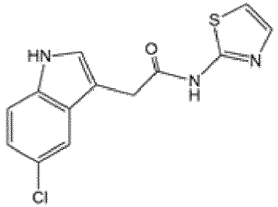 |

**[TABLE 2c]**

| **Compound** | **Structure** |
|---|---|
| 104 | 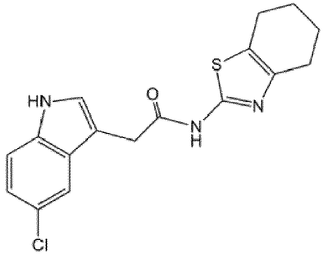 |
| 109 | 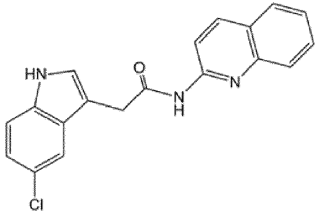 |

**[TABLE 2d]**

| **No. of compound** | **Molecular weight** | **No. of compound** | **Molecular weight** |
|---|---|---|---|
| 6 | 358.23 | 88 | 285.73 |
| 5 | 344.21 | 44 | 321.40 |
| 8 | 378.65 | 47 | 355.84 |
| 43 | 307.37 | 54 | 321.40 |
| 40 | 341.81 | 60 | 339.39 |
| 23 | 303.72 | 56 | 355.84 |
| 26 | 338.16 | 64 | 325.36 |
| 71 | 340.37 | 41 | 341.81 |
| 68 | 374.82 | 99 | 291.76 |
| 81 | 319.19 | 109 | 335.79 |
| 78 | 353.63 | 104 | 345.85 |
| 2 | 362.20 | | |

### Example: Measurement of activity of compound - experimental protocol

### 1. Search and preparation of compound

In order to confirm target specificity of the prepared compounds, evaluation was performed by the following method.

After recovering HepG2 under culture in DMEM-fetal bovine serum (FBS) 10% medium and then confirming that the survival rate is 97% or more through trypan blue staining, the recovered product was centrifuged at a speed of 1200 rpm for 5 minutes at room temperature, and the cells were prepared by resuspending the cells in DMEM-fetal calf serum 10% medium at 3 × 10⁵ cells/ml. Thereafter, the cells were dispensed onto a 60 mm dish by 3 ml, and each dish was treated with 50 µl of compounds at a concentration of 5 µM diluted in DMEM medium, and then cultured in a cell incubator (5% CO₂ incubator) for 24 hours. As a control, 50 µl of 0.05% dimethylsulfoxide (DMSO)/DMEM medium was used for treatment.

The cultured cells were recovered to prepare an mRNA sample. Specifically, mRNA was extracted from the recovered cells by a phenol-chloroform precipitation method using Trizol reagent (Invitrogen, Cat No. 15596018). From the isolated RNA, cDNA was synthesized by reverse transcription, and the expression of CYP1A1 was confirmed through a real-time polymerase chain reaction (PCR) using iQ SYBR-Green Supermix (Bio-rad) in a CFX96 (Bio-rad) detection system. Relative values of the enzyme expression levels were compared by ΔΔct method using GAPDH as a control enzyme. Herein, one (1) fold was set using the control.

The real-time polymerase chain reaction was performed under the conditions of 45 cycles at an annealing temperature of 58 °C, wherein the following primer sequences were used.

Human CYP1A1 forward, 5'-CAC CCT CAT CAG TAA TGG TCA GA-3' (SEQ ID NO: 1) and reverse, 5'-AAC GTG CTT ATC AGG ACC TC-3' (SEQ ID NO: 2); Human GAPDH forward, 5'-TGA TGA CAT CAA GAA GGT GG-3' (SEQ ID NO: 3) and reverse, 5'-TTA CTC CTT GGA GGC CAT GT-3' (SEQ ID NO: 4).

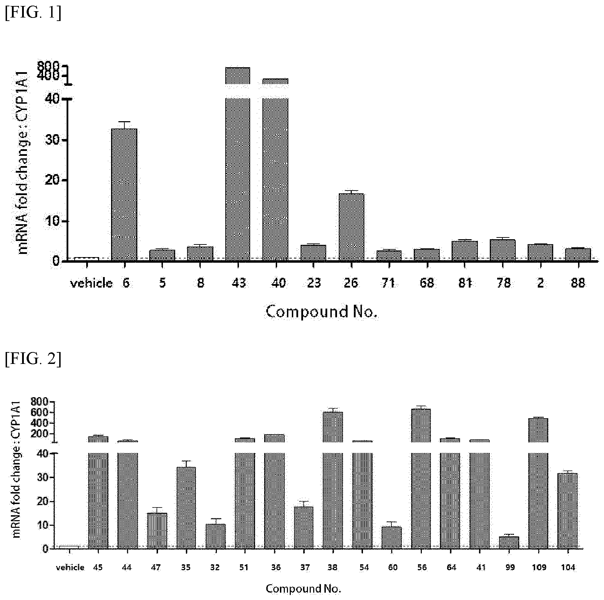

As a result, it could be seen that the CYP1A1 expression level was higher than that of the control (vehicle) in the tested compounds, and it could further be seen that CYP1A1 expression was significantly induced (FIGS. 1 and 2).

### 2. Inhibitory effects of production of inflammatory factor IL-6

In order to assess the inhibitory effects of production of the macrophage IL-6 by the compounds according to the present invention, the following experiments were implemented.

After recovering THP-1 under culture in RPMI-fetal bovine serum (FBS) 10% + 2-ME (mercaptoethanol) medium and confirming that the survival rate is 97% or more through trypan blue staining, the recovered product was centrifuged at a speed of 1200 rpm for 5 minutes at room temperature, and the cells were prepared by resuspending the cells in RPMI-fetal calf serum 10% + 2-ME medium at 5 × 10⁵ cells/ml. Thereafter, the cells were dispensed by 500 µl onto a 24-well plate (3 wells per sample), and then, 0.5 µl of PMA was added to 200 ng/ml in each well, and each well was treated with 10 µl of compounds at a concentration of 5 µM diluted in RPMI + 2ME medium. After incubation in a cell incubator (5% CO₂ incubator) for 48 hours, 5 µl of LPS dissolved in dPBS was added at 100 ng/ml for treatment, followed by culturing the same in a cell incubator (5% CO₂ incubator) for 24 hours.

As a control, 10 µl of 0.05% dimethylsulfoxide (DMSO)/RPMI medium was used for treatment. The culture medium of the cultured cells was recovered using a new microtube, and the cells were recovered with 1 ml of Trizol (invitrogen) and stored at - 80 °C. The sample was diluted by 1/5 by putting 10 µl of the recovered medium and 40 µl of assay diluent buffer into a FACS tube (BD falcon), followed by vortexing capture beads in each sample, and then, 1 µl of the vortexed solution and capture bead diluent. Then, 49 µl of capture bead diluents were added to prepare 50 µl of a capture bead solution for each sample. After mixing the capture bead solution by vortexing, 50 µl of the capture bead solution was put into a FACS tube containing each sample, vortexed again, and left at room temperature for 1 hour.

After 1 hour, 1 µl of PE detection reagent and 49 µl of PE detection reagent diluent were added to prepare 50 µl of PE detection solution for each sample. After vortexing, 50 µl of PE detection solution for each sample was added to the FAC tube containing the capture bead solution and the sample. After vortexing, the FACS tube was left at room temperature for 1 hour. After 1 hour, 1 ml of CBA wash buffer was added to each tube, centrifuged at 400 g for 5 minutes, and the supernatant was removed. After vortexing gently, 150 µl of fixation buffer was added, vortexed gently, followed by analysis using a flow cytometry.

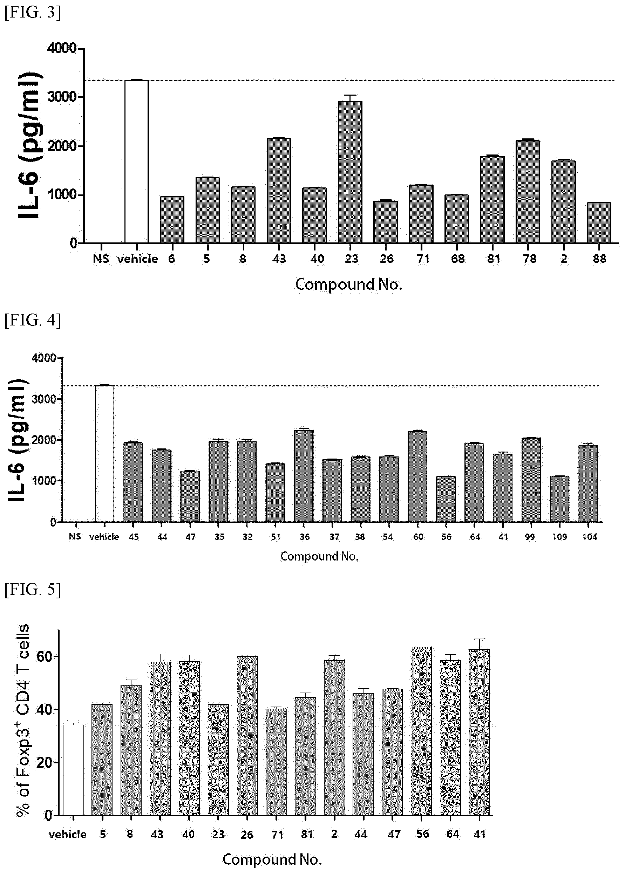

As a result, as shown in FIGS. 3 and 4, IL-6 production by THP-1 through LPS stimulation was significantly reduced by treatment with the compound. Specifically, all of the compounds of the present invention showed low results compared to the results in the control (vehicle), and this means that the compounds effectively inhibit the production of IL-6.

### 3. Regulatory T cell production effects

In order to examine effects of inducing immune tolerance by the compounds according to the present invention, *in vitro* regulatory T cell (Foxp3+ Treg) production experiments were conducted as follows.

Human peripheral blood (AllCells) and phosphate buffered saline (PBS) were mixed in a ratio of 1:1 to prepare a mixture, followed by allowing the mixture to slowly rise so as not to be mixed into the upper layer of Histopaque (Sigma). After centrifugation at 350 g for 20 minutes, only the monocyte layer in the middle layer was collected and washed with HBSS (Hanks' Balanced Salt Solution, Gibco^{®}). After washing with MACS buffer (Miltenyi Biotec) once more to obtain T-cells, positive selection (autoMACS seperator, Miltenyi Biotec) was performed with CD4 Microbeads (Miltenyi Biotec). The T-cells collected by the above method were prepared by resuspending the cells in RPMI-fetal bovine serum (FBS) 10% + 2-ME (mercaptoethanol) medium at 5 × 10⁵ /ml. For T-cell activation, 10 µg/ml anti-CD3 (eBioscience^{™}) was dispensed by 150 µl into a 48-well plate, reacted in a cell incubator (37 °C, 5% CO₂ incubator) for 3 hours, and washed with phosphate buffered saline to prepare the plate. 250 µl of resuspended T-cells was dispensed onto the prepared plate, and each well was treated with 2 µg/ml of anti-CD28 (eBioscience^{™}), 5 ng/ml of TGFβ-1 (R&D systems), and 50 U/ml of IL-2 (Miltenyi Biotec). Each 5 µl of compounds at a concentration of 2.5 µM diluted in RPMI + 2ME medium was used for treatment, followed by culturing the same in a cell incubator (37 °C, 5% CO ₂ incubator) for 7 days. As a control, 5 µl of 0.05% dimethylsulfoxide (DMSO)/RPMI medium was used for treatment. After 7 days, in order to confirm effects of producing regulatory T cells, the cultured cells were recovered to determine the presence of Foxp3 protein.

The recovered cells were placed in a 5 ml FACS tube (BD Falcon) and washed with 1 ml of phosphate buffered saline. The cells were resuspended in 0.1 ml of FACS buffer (0.1% NaN₃, 1% FBS) and treated with 1 µg of human immunoglobulin G (Human IgG, Sigma) to prevent non-specific binding of the antibody. After reacting at 4 °C for 15 minutes, the cells were washed with FACS buffer. Then, 1 ml of Fixation/Permeabilization solution (eBioscience^{™}) was added to the FACS tube containing each sample, followed by reaction at 4 °C for 1 hour. Thereafter, the product was washed twice with a Permeabilization buffer (eBioscience^{™}). Then, 0.25 µg of Foxp3 monoclonal antibody (eBioscience^{™}) was used for treatment, followed by staining the sample for 4 to 30 minutes. The cells were washed twice with the permeabilization buffer, suspended in 0.3 ml of FACS buffer, and measured by flow cytometry.

As a result, it was confirmed that the generation of Foxp3+ regulatory T cells was promoted by treatment with the tested compounds Nos. 5, 8, 43, 40, 23, 26, 71, 81, 2, 44, 47, 56, 64 and 41 (see FIG. 5). Through this, it could be seen that the compounds effectively induce the production and proliferation of regulatory T cells.

### 4. Confirmation of treatment effects of inflammatory bowel diseases

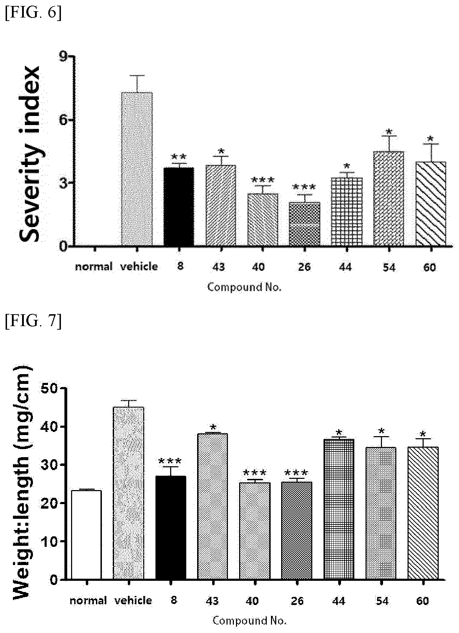

In order to investigate therapeutic effects of the compounds according to the present invention on inflammatory bowel disease, the inflammatory bowel disease was induced in C57BL/6 mice, and the compounds (Nos. 8, 43, 40, 26, 44, 54 and 60) were administered to evaluate the efficacy as follows (FIGS. 6 to 7).

On day 0 of the experiment, a 1.5% DSS solution prepared by dissolving DSS (Dextran sulfate sodium, MP biomedicals, Cat No. 160110) in 1.5% sterile distilled water was given for drinking to C57BL/6 mice (8 weeks old, female, 18 ± 2 g) for 7 days. The 1.5% DSS solution was changed at an interval of 2 days. Sterile distilled water was provided for drinking from the 8th day of the experiment. Body weight and severity index were measured at an interval of 2 days from the 0th day of the experiment in order to confirm the onset of inflammatory bowel disease.

20 mg/kg of the compound per mouse was completely dissolved in DMSO corresponding to 10% (v/v) of the administered dose, and then diluted in a cremophor EL-phosphate buffered saline mixture in order to produce the final DMSO:Cremophor EL:phosphate buffered saline (1:1:8, v/v/v), followed by oral administration of 200 µl daily for a total of 10 times from the 2nd to 11th days of the experiment. An inflammatory bowel disease severity index was visually observed and recorded at an interval of 2 days according to a severity index system classified into 0 to 10 levels.

Inflammatory bowel disease symptoms were evaluated by summing the scores of three items according to the following items (Table 3).

**[TABLE 3]**

| Score | Symptoms | | |
|---|---|---|---|
| | Watery of excrement | Melena state | Reduction of Body weight |
| 0 | Normal form | Normal form | Normal |
| 1 | Slightly loose feces | Brown excrement | 5-10% decrease |
| 2 | Loose feces | Redish brown excrement | 11-15% decrease |
| 3 | Diarrhea | Melena | 16-20% decrease |
| 4 | - | - | > 20% decrease |

As a result of the analysis, it was confirmed that the body weight of the solvent control started to decrease from the 6th day of the experiment, decreased by 10% or more on the 10th day of the experiment, and 100% of enteritis was induced along with an increased severity index of 5 or more. The mice in the solvent control showed a severity index of 7.29 ± 2.29 on the 10th day of the experiment when the severity index reached the maximum. On the other hand, the experimental group administered with 20 mg/kg of the compound No. 8, 43, 40, 26, 44, 54 or 60 of the present invention showed statistically significant therapeutic effects compared to the solvent control on the 10th day of the experiment. Further, comparison of the colon weight:length ratio on the 15th day of the experiment (weight:length ratio, mg/cm) demonstrated that intestinal inflammation could be significantly suppressed in terms of morphology (compared to the solvent control: ^{∗}, p<0.05; ^{∗∗}, p<0.01; ^{∗∗∗}, p<0.001, see FIGS. 6 and 7). Specifically, the compounds Nos. 8, 43, 40, 26, 44, 54 and 60 of the present invention showed excellent anti-inflammatory effects when administered in an amount of 20 mg/kg.

On the 15th day of the experiment, the colon of the mouse was excised to prepare an mRNA sample. In order to extract mRNA, the colon tissue was ground with a homogenizer to acquire a homogeneous suspension. From the homogeneous suspension, mRNA was extracted by a phenol-chloroform sedimentation method using an easy-spin^{™} (DNA free) total RNA extraction kit (Intron biotechnology, Cat No. 17221). From the isolated RNA, cDNA was synthesized by reverse transcription, followed by confirming the expression of inflammatory cytokines through real-time polymerase chain reaction (PCR) using iQ SYBR-Green Supermix (Bio-rad) in the CFX96 (Bio-rad) detection system. Relative values of the enzyme expression levels were compared by the ΔΔct method using GAPDH as a control enzyme. Herein, one (1) fold was set using the normal mouse colon as a control.

The real-time polymerase chain reaction was implemented under the conditions of 45 cycles at an annealing temperature of 58 °C, and the following primer sequences were used.
Mouse IL-1β forward, 5'-CTC GTG CTG TCG GAC CCA TAT-3' (SEQ ID NO: 5) and reverse, 5'-TTG AAG ACA AAC CGC TTT TCC A-3' (SEQ ID NO: 6);
Mouse IL-6 forward, 5'-CAT GTT CTC TGC GAA ATC GTG G-3' (SEQ ID NO: 7) and reverse, 5'-AAC GCA CTA GGT TTG CCG AGT A-3' (SEQ ID NO: 8);
Mouse IL-17A forward, 5'-TTT AAC TCC CTT GGC GCA AAA-3' (SEQ ID NO: 9) and reverse, 5'-CTT TCC CTC CGC ATT GAC AC-3' (SEQ ID NO: 10);
Mouse TNF-α forward, 5'-CCA CAC CGT CAG CCG ATT TG-3' (SEQ ID NO: 11) and reverse, 5'-CAC CCA TTC CCT TCA CAG AGC-3' (SEQ ID NO: 12);
Mouse IL-10 forward, 5'-CAA GGC AGT GGA GCA GGT GAA-3' (SEQ ID NO: 13) and reverse, 5'-CGG AGA GAG GTA CAA ACG AGG TT-3' (SEQ ID NO: 14);
Mouse Foxp3 forward, 5'-CCC ATC CCC AGG AGT CTT G-3' (SEQ ID NO: 15) and reverse, 5'-ACC ATG ACT AGG GGC ACT GTA-3' (SEQ ID NO: 16);
Mouse GAPDH forward, 5'-TTC ACC ACC ATG GAG AAG GC-3' (SEQ ID NO: 17) and reverse, 5'-GGC ATG GAC TGT GGT CAT GA-3' (SEQ ID NO: 18).

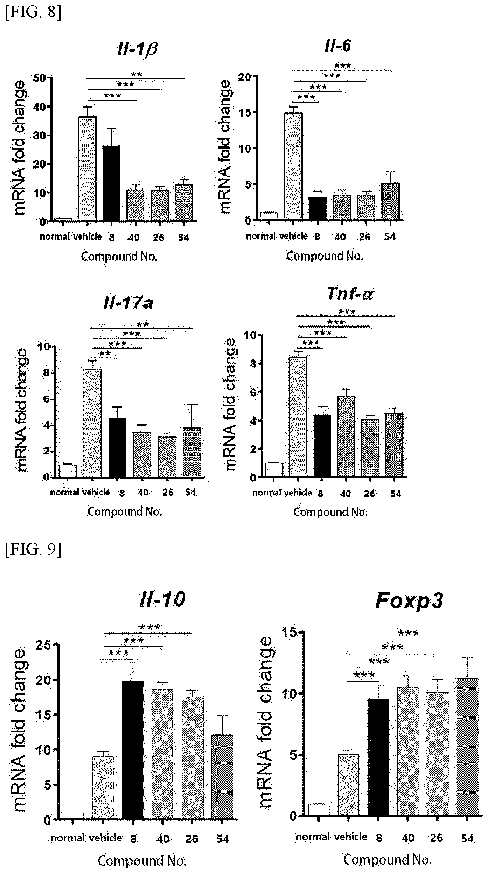

The expression levels of the inflammatory cytokines IL-1β, IL-6, IL-17A, and TNF-α in colon lesions were significantly reduced compared to the solvent control by administration of the compound No. 8, 40, 26 or 54 (compared to the solvent control ^{∗∗}, p <0.01; ^{∗∗∗}, p<0.001, see FIG. 8). Further, the expression levels of the immunomodulatory factors IL-10 and Foxp3 in the colon lesion were significantly increased compared to the solvent control by administration of the compound No. 8, 40, 26, or 54 (compared to the control ^{∗∗∗}, p<0.001, see FIG. 9). From these results, it could be seen that the compound Nos. 8, 40, 26 and 54 of the present invention could significantly reduce the expression of inflammatory factors in the intestine while significantly increasing the expression of immunomodulatory factors in the intestine.

In order to investigate mucosal healing effects of the compounds according to the present invention, inflammatory bowel disease was induced in C57BL/6 mice, and a degree of recovery of intestinal epithelial barrier integrity was assessed by administering the compounds (Nos. 40, 26, 54) as follows.

On day 0 of the experiment, a 1.5% DSS solution prepared by dissolving 1.5% DSS in sterile distilled water was given for drinking to C57BL/6 mice (8 weeks old, female, 18 ± 2 g) for 7 days. The 1.5% DSS solution was changed at an interval of 2 days. Sterile distilled water was provided for drinking from the 8th day of the experiment. Body weight and severity index were measured at an interval of 2 days from the 0th day of the experiment, so as to confirm the onset of inflammatory bowel disease.

In the compound No. 40, 26 or 54 administration group according to the present invention, 20 mg/kg of the compound per mouse was completely dissolved in DMSO corresponding to 10% (v/v) of the administered dose, and then, was diluted in a cremophor EL-phosphate buffered saline mixture to prepare the final DMSO:Cremophor EL:phosphate buffered saline (1:1:8, v/v/v), followed by oral administration with 200 µl of the prepared solution daily for a total of 8 times from the 2nd day to 9th day of the experiment.

One day before FITC-dextran administration, a mouse was deprived of water overnight. On the 10th day of the experiment, 600 mg/kg of FITC-dextran (Fluorescein isothiocyanate-dextran, Sigma Aldrich, Cat No. FD40) was diluted in a phosphate buffered saline and administered orally to the mouse at 200 µl once. 4 hours after oral administration, fluorescence was measured in the serum extracted from the heart (fluorometer, excitation 485-490 nm, emission 528-530 nm).

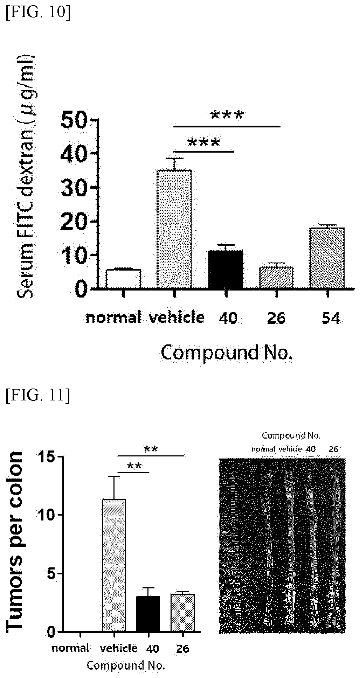

Serum FITC-dextran was significantly reduced compared to the solvent control by administration of the compound No. 40, 26 or 54 (compared to the solvent control: ^{∗∗∗}, p<0.001, see FIG. 10). From this result, it could be seen that the compounds Nos. 40, 26, and 54 of the present invention exhibit significant mucosal healing effects.

As such, the compounds 8, 43, 40, 26, 44, 54 and 60 of the present invention have therapeutic effects when orally administered in the mouse model with inflammatory bowel disease. Therefore, these compounds may propose a useful treatment strategy as novel orally administered therapeutic agents for inflammatory bowel disease.

### 5. Confirmation of effects of preventing inflammation-induced colon cancer

In order to investigate the effects of the compounds according to the present invention to prevent inflammation-induced colon cancer, medical efficacy was evaluated by administering the compound Nos. 40 and 26, respectively, to the AOM/DSS-induced colon cancer mouse model in C57BL/6 mice as follows (FIG. 11). Azoxymethane (AOM) as a carcinogen cannot cause colon cancer when administered alone to mice. However, when inflammation is added using DSS, colon cancer occurs.

AOM (Sigma Aldrich, Cat No. A5486) was diluted with physiological saline to a concentration of 10 mg/kg, and then administered intraperitoneally three times at an interval of 7 days (Experiment day 0, 7, 14th). On the 7th day of the experiment, a 1.5% DSS solution prepared by dissolving 1.5% DSS in sterile distilled water was given for drinking to C57BL/6 mice (8 weeks old, female, 18 ± 2 g) for 7 days. Further, the 1.5% DSS solution was changed at an interval of 2 days. Sterile distilled water was provided for drinking from the 8th day of the experiment.

In the administration groups of compound Nos. 40 and 26 according to the present invention, respectively, 20 mg/kg of the compound per mouse was completely dissolved in DMSO corresponding to 10% (v/v) of the administered dose, and then, was diluted in a cremophor EL-phosphate buffered saline mixture to prepare the final DMSO:Cremophor EL:phosphate buffered saline (1:1:8, v/v/v), followed by oral administration with 200 µl of the prepared solution daily for a total of 14 times from the 7th day to 21st day of the experiment. At a time when the body weight of the solvent control decreased by 15% compared to the 56th day of the experiment, colon cancer preventing effects of the compound was confirmed.

On the 93rd day of the experiment, the colon of the mouse was excised to confirm the occurrence of tumor in the colon. The number of tumors in the colon was 11.33 ± 4.33 in the solvent control, 3.00 ± 2.00 in the case of compound No. 40, and 3.17 ± 1.17 in the case of compound No. 26, such that the number of tumors were significantly reduced compared to the solvent control by administration of the compound Nos. 40 and 26 (compared to the solvent control: ^{∗∗}, p<0.01, see FIG. 11).

As such, the compounds Nos. 40 and 26 of the present invention have inhibitory effects on the occurrence of inflammation-induced colon cancer. Therefore, these compounds may propose a useful treatment strategy as novel orally administered therapeutic agents for inflammatory bowel disease with colon cancer prevention effects.

### 6. Confirmation of multiple sclerosis treatment effects

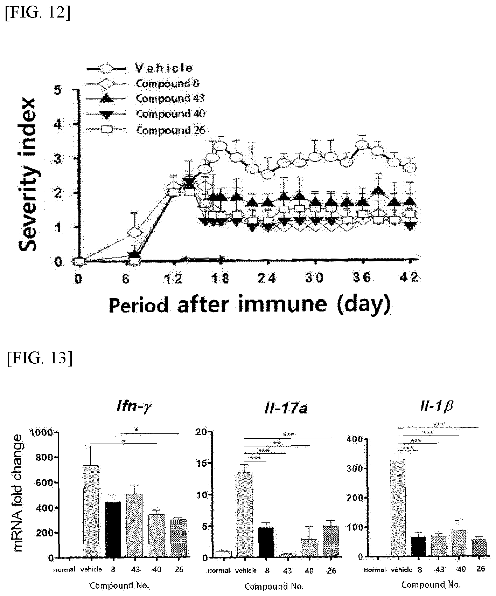

In order to investigate therapeutic effects of the compounds according to the present invention on multiple sclerosis, autoimmune encephalomyelitis (EAE) was induced in C57BL/6 mice and medical efficacy was evaluated by administering the compounds (8, 43, 40, 26) (FIGS. 12 to 14).

On day 0, myelin oligodendrocyte glycoproteins 35-55 (MOG 35-55, Peptron) (200 µg), heat-killed mycobacterium tuberculosis (Difco, Cat No. 231141) (500 µg), and adjuvant (Complete Freund's adjuvant, Sigma Aldrich, Cat No. F5506) were mixed together and then submerged for 7 minutes. After subcutaneous injection of 100 µl of the submerged peptide into both flanks of each of C57BL/6 mice (7 weeks old, female, 17 ± 2 g), 100 µl of pertussis toxin (Sigma Aldrich, Cat No. P2980) (200 ng) was administered intravenously to the tail.

On the 2nd day of the experiment, the same amount of pertussis toxin was administered intravenously. The mice were checked for immersion leaking from the injected site, and visually observed from the 7th day of the experiment in order to confirm the onset of multiple sclerosis.

In the groups treated with the compounds Nos. 8, 43, 40 and 26, 20 mg/kg of the compound per mouse was completely dissolved in DMSO corresponding to 10% (v/v) of the administered dose, and then, was diluted in a cremophor EL-phosphate buffered saline mixture to prepare the final DMSO:Cremophor EL:phosphate buffered saline (1:1:8, v/v/v), followed by administering intraperitoneally 200 µl of the prepared solution daily for a total of 6 times from the 12th day to 17th day of the experiment. The multiple sclerosis index was visually observed and recorded at an interval of 2 days from the 7th day of the experiment in the severity index system classified into 0-5 stages, and autoimmune encephalomyelitis symptoms were indexed according to the following items (see Table 4).

**[TABLE 4]**

| Score | Symptoms |
|---|---|
| 0 | No symptom |
| 1 | Tail enervated |
| 2 | Tail enervated, and hindlimb weaken |
| 3 | Hindlimb paralysis |
| 4 | Hindlimb paralysis, and forelimb weaken |
| 5 | Death or being near death |

As a result of the analysis, it was confirmed that all experimental groups developed multiple sclerosis from the 7th day of the experiment, and 100% of the acute reactions with a severity index of 3.0 or more were induced on the 18th day of the experiment.

Mice in the solvent control had the severity index of 3.33 ± 0.17 on the 18th day of the experiment, which is the acute reaction period, and 3.33 ± 0.17 on the 36th day of the experiment, which is the chronic reaction period. Further, the solvent control showed a relapse-remitting pattern and a high severity index throughout the experiment.

On the other hand, the severity index of the compound treatment group was: on the 18th day of the experiment, 1.17 ± 0.56 in the compound No. 8 treatment group, 1.83 ± 0.17 in the compound No. 43 treatment group, 1.17 ± 0.22 in the compound No. 40 treatment group, and 1.33 ± 0.56 in the compound No. 26 treatment group; and, on the 36th day of the experiment, 1.17 ± 0.56 in the compound No. 8 treatment group, 2.00 ± 0.33 in the compound No. 43 treatment group, 1.33 ± 0.62 in the compound No. 40 treatment group, 1.33 ± 0.22 in the compound No. 26 treatment group, which demonstrated alleviated acute response and chronic response treatment effects, as compared to the solvent control.

Further, it could be confirmed that the groups treated with the compounds Nos. 8, 43, 40 and 26 had statistically significant treatment effects compared to the solvent control from the 16th day of the experiment, and even after the administration was stopped, the statistically significant treatment effects were maintained compared to the solvent control (compared to the solvent control: ^{∗∗∗}, p<0.001, see FIG. 12). From the above results, it could be seen that, 20 mg/kg of administration to the mouse exhibits excellent initial treatment and continuous recurrence prevention effects.

On the 42nd day of the experiment, the spinal cord of the mouse was excised to prepare an mRNA sample. In order to extract mRNA, the spinal cord tissue was ground with a homogenizer to obtain a homogeneous suspension. The mRNA was extracted from the homogeneous suspension by a phenol-chloroform precipitation method using Trizol reagent (Invitrogen, Cat No. 15596018). Then, cDNA was synthesized from the isolated RNA by reverse transcription, and the expression of inflammatory cytokines was investigated through real-time polymerase chain reaction (PCR) using iQ SYBR-Green Supermix (Bio-rad) in the CFX96 (Bio-rad) detection system. Relative values of the enzyme expression levels were compared by the ΔΔct method using GAPDH as a control enzyme. Herein, one (1) fold was set using the WT mouse spinal cord as a control.

The real-time polymerase chain reaction was implemented under the conditions of 45 cycles at an annealing temperature of 58 °C, and the following primer sequences were used.
Mouse IFN-γ forward, 5'-ATG AAC GCT ACA CAC TGC ATC-3" (SEQ ID NO: 19) and reverse, 5'-CCA TCC TTT TGC CAG TTC CTC-3" (SEQ ID NO: 20);
Mouse IL-17A forward, 5'-TTT AAC TCC CTT GGC GCA AAA-3" (SEQ ID NO: 21) and reverse, 5'-CTT TCC CTC CGC ATT GAC AC-3" (SEQ ID NO: 22);
Mouse IL-1β forward, 5'-CTC GTG CTG TCG GAC CCA TAT-3" (SEQ ID NO: 23) and reverse, 5'-TTG AAG ACA AAC CGC TTT TCC A-3" (SEQ ID NO: 24);
Mouse GAPDH forward, 5'-TTC ACC ACC ATG GAG AAG GC-3" (SEQ ID NO: 25) and reverse, 5'-GGC ATG GAC TGT GGT CAT GA-3" (SEQ ID NO: 26);
Mouse IL-10 forward, 5'-CAA GGC AGT GGA GCA GGT GAA-3' (SEQ ID NO: 27) and reverse, 5'-CGG AGA GAG GTA CAA ACG AGG TT-3" (SEQ ID NO: 28);
Mouse Foxp3 forward, 5'-CCC ATC CCC AGG AGT CTT G-3" (SEQ ID NO: 29) and reverse, 5'-ACC ATG ACT AGG GGC ACT GTA-3" (SEQ ID NO: 30).

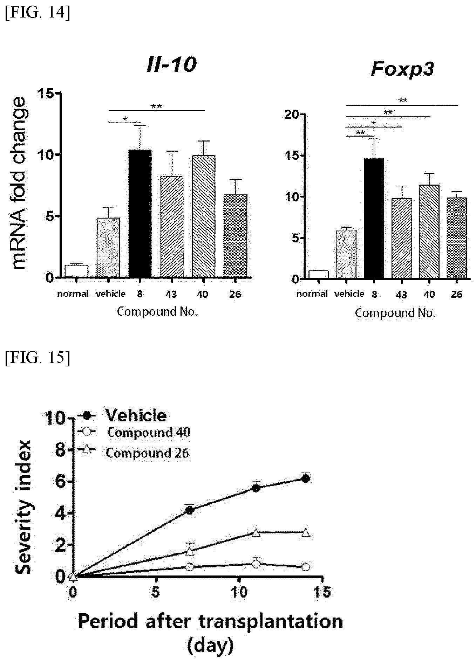

Further, the expression levels of inflammatory cytokines IFN-γ, IL-17A, and IL-1β, respectively, in the spinal cord lesion were significantly reduced compared to the solvent control by administration of the compound Nos. 8, 43, 40 and 26 (compared to the solvent control: ^{∗}, p<0.05; ^{∗∗}, p<0.01; ^{∗∗∗}, p<0.001, see FIG. 13). The expression levels of the immunomodulatory factors IL-10 and Foxp3 in spinal cord lesions were significantly increased compared to the solvent control by administration of the compound Nos. 8, 43, 40 and 26 (compared to the solvent control: ^{∗}, p<0.05; ^{∗∗}, p<0.01, see FIG. 14).

As such, the compound Nos. 8, 43, 40 and 26 of the present invention have therapeutic efficacies in the multiple sclerosis mouse model, and even after the administration was stopped, the effects of preventing recurrence are continuously maintained. Therefore, these compounds may propose a useful treatment strategy as novel orally administered therapeutic agents for multiple sclerosis.

### 7. Confirmation of graft-versus-host disease treatment effects

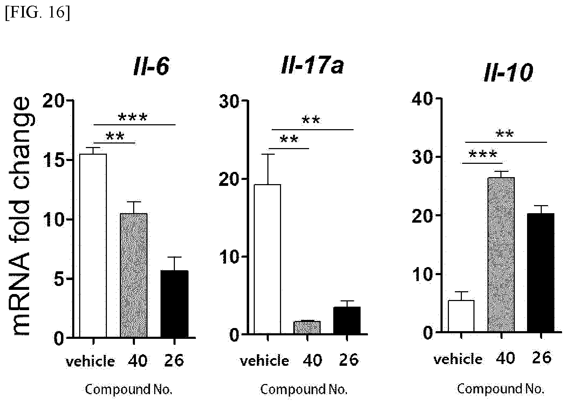

In order to investigate the inhibitory effects of the compounds of the present invention on graft-versus-host disease (GVHD), acute graft-versus-host disease was induced by allogeneic bone marrow transplantation in C57BL/6 mice as follows, and the compound (No. 40 or 26) was administered to evaluate its efficacy (FIGS. 15 and 16).

The spleen of Balb/c IFN-γ knockout mouse (8 to 12 weeks old, female, 18 ± 3 g) was excised, pulverized by adding RPMI medium, and then passed through a 40 µm cell strainer (BD Falcon), thereby obtaining a single cell suspension. The single cell suspension was centrifuged (1200 rpm, 5 minutes), and after discarding the supernatant, 1 ml of ACK (ammonium chloride/potassium bicarbonate) lysis buffer (0.15 M NH4Cl, 1 mM KHCO3, 0.1 mM Na2EDTA) was added, followed by stirring for 1 minute and then washing the same with RPMI medium.

After centrifugation, the cell suspension was reacted on mouse CD90.2 microbeads (Miltenyi Biotec, Cat No. 130-121-278) at 4 °C for 20 minutes. After complementation of the reaction, the cell suspension was centrifuged, washed with 10 ml of autoMACS^{®} Running Buffer (Miltenyi Biotec, Cat No. 130-091-221), and then resuspended with 3 ml of autoMACS^{®} Running Buffer. Then, CD90.2⁺ T cells were obtained from the cell suspension using Auto MACS pro (Miltenyi Biotec) (positive selection). To obtain bone marrow cells to be transplanted together with the obtained CD90.2⁺ T cells, both femurs and tibias of wild-type Balb/c mice (8-12 weeks old, female, 18 ± 3 g) were aseptically acquired. End portions of the femur and tibia were cut, and the bone marrow was extracted by perfusion of RPMI medium to the bone tissue with a syringe (femur 21G, tibia 26G). The extracted bone marrow was passed through a 40 µm cell strainer to obtain a single cell suspension.

The bone marrow single cell suspension was centrifuged, and after discarding the supernatant, 500 µl of ACK lysis buffer was added, followed by stirring for 30 seconds and washing the solution with RPMI medium. After centrifugation, the suspension was reacted on the mouse CD90.2 microbeads at 4 °C for 20 minutes. After complementation of the reaction, the cell suspension was centrifuged, washed with 10 ml of autoMACS^{®} Running Buffer, and then resuspended with 3 ml of autoMACS^{®} Running Buffer. Then, CD90.2⁻ T cell-depleted bone marrow cells (TCD-BMs) were obtained from the cell suspension through Auto MACS pro (negative selection). The obtained IFN-γ knockout CD90.2⁺ T cells and normal TCD-BMs were washed with phosphate buffered saline. T cells were prepared by resuspending the same in phosphate buffered saline at 1 × 10⁷/ml, while TCD-BM was prepared by resuspending the same in phosphate buffered saline at 5 × 10⁷/ml.

Normal C57BL/6 mice (9 to 11 weeks old, female, 19 ± 3 g) were irradiated with 850 cGy of radiation divided at an interval of 3 hours by using a radiation irradiator. A graft prepared by mixing the prepared CD90.2⁺ T cells and TCD-BM at a ratio of 1:1 was injected through the tail vein of C57BL/6 mice at a rate of 100 µl. In the compound No. 40 or 26 administration group according to the present invention, 20 mg/kg of the compound per mouse was completely dissolved in DMSO corresponding to 10% (v/v) of the administered dose, and then was diluted in a cremophor EL-phosphate buffered saline mixture to prepare the final DMSO:Cremophor EL:phosphate buffered saline (1:1:8, v/v/v), followed by administering the solution intraperitoneally daily for a total of 6 times from 4 to 9 days after transplantation by 200 µl. The graft-versus-host disease severity index was evaluated at an interval of 2 days by visual observation in a severity index system that was classified into a total of 10 points with 0 to 2 points for each item including reduction of body weight, hair condition, posture, activity and skin change.

As a result of the analysis, 100% of graft-versus-host disease was induced in the mice of the solvent control with a severity index of 4.12 ± 1.20 on the 7th day after transplantation. Further, on the 14th day after transplantation, the severity indexes were: 6.20 ± 1.20 in the solvent control; 0.60 ± 0.60 in the compound No. 40 treatment group; and 2.80 ± 0.80 in the compound No. 26 treatment group, which demonstrated significantly alleviated graft-versus-host disease and treatment effects compared to the solvent control (compared to the solvent control: ^{∗∗∗}, p<0.001, see FIG. 15).

On the 15th day of the experiment, the lungs of the mice were excised to prepare an mRNA sample. In order to extract mRNA, the spinal cord tissue was ground with a homogenizer to obtain a homogeneous suspension. The mRNA extracted from the homogeneous suspension by a phenol-chloroform precipitation method using Trizol reagent (Invitrogen, Cat No. 15596018). Then, cDNA was synthesized from the isolated RNA by reverse transcription, and the expression of inflammatory cytokines was confirmed through real-time polymerase chain reaction (PCR) using iQ SYBR-Green Supermix (Bio-rad) in the CFX96 (Bio-rad) detection system. Relative values of the enzyme expression levels were compared by the ΔΔct method using GAPDH as a control enzyme. Herein, one (1) fold was set using the normal mouse spinal cord as a control.

The real-time polymerase chain reaction was implemented under the conditions of 45 cycles at an annealing temperature of 58 °C, and the following primer sequences were used.

Mouse IL-17A forward, 5'-TTT AAC TCC CTT GGC GCA AAA-3' (SEQ ID NO: 31) and reverse, 5'-CTT TCC CTC CGC ATT GAC AC-3' (SEQ ID NO: 32);

Mouse IL-6 forward, 5'-CAT GTT CTC TGC GAA ATC GTG G-3' (SEQ ID NO: 33) and reverse, 5'-AAC GCA CTA GGT TTG CCG AGT A-3' (SEQ ID NO: 34);

Mouse IL-10 forward, 5'-CAA GGC AGT GGA GCA GGT GAA-3' (SEQ ID NO: 35) and reverse, 5'-CGG AGA GAG GTA CAA ACG AGG TT-3' (SEQ ID NO: 36);

Mouse GAPDH forward, 5'-TTC ACC ACC ATG GAG AAG GC-3' (SEQ ID NO: 37) and reverse, 5'-GGC ATG GAC TGT GGT CAT GA-3' (SEQ ID NO: 38).

The expression levels of the inflammatory cytokines IL-6 and IL-17A in lung tissue were significantly reduced compared to the solvent control by administration of the compound Nos. 40 and 26. Further, the expression level of the immunomodulatory factor IL-10 in lung tissue was significantly increased compared to the solvent control by administration of the compound Nos. 40 and 26 (compared to the solvent control: ^{∗∗}, p<0.01; ^{∗∗∗}, p<0.001, see FIG. 16).

As such, the compound Nos. 40 and 26 of the present invention have therapeutic efficacy in the mouse model with graft-versus-host disease, and even after the administration was stopped, the therapeutic efficacy is continuously maintained due to an increase in immunomodulatory factors. Therefore, these compounds may propose a useful treatment strategy as novel orally administered therapeutic agents for graft-versus-host disease.

## Claims

1. A compound represented by Formula 1 below, a stereoisomer or a pharmaceutically acceptable salt thereof: 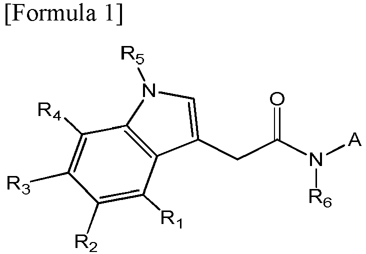 (wherein R₁ to R₄ are each independently hydrogen or halogen, R₅ and R₆ are each independently hydrogen or C₁ - C₅ alkyl,
A is a single or double cyclic group of C₅ - C₁₂,
each ring of the cyclic group is substituted with 1 to 3 heteroatoms, and
the cyclic group is substituted with halogen, C₁-C₅ alkyl or C₁-C₅ alkoxy).

2. The compound, the stereoisomer or the pharmaceutically acceptable salt thereof according to claim 1, wherein A is selected from the group consisting of the following cyclic groups: 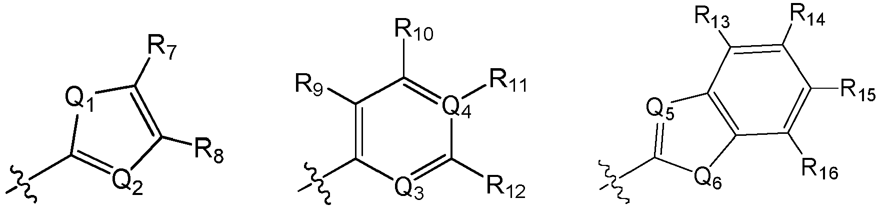 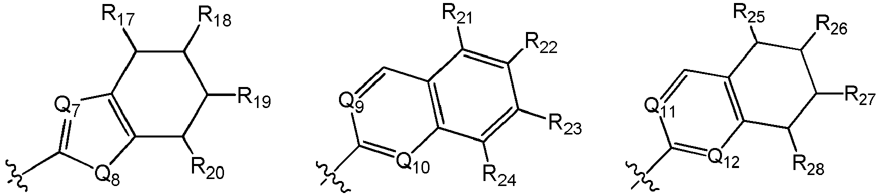 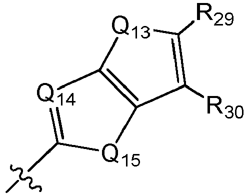 (wherein Q₁ to Q₁₅ are each independently C, N or S, and R₇ to R₃₀ are each independently hydrogen, halogen, C₁-C₃ alkyl or C₁-C₃ alkoxy, and if Q₄ is N, R₁₁ is absent).

3. The compound, the stereoisomer or the pharmaceutically acceptable salt thereof according to claim 1, wherein A is selected from the group consisting of the following cyclic groups: 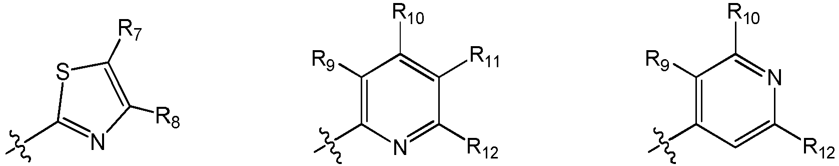 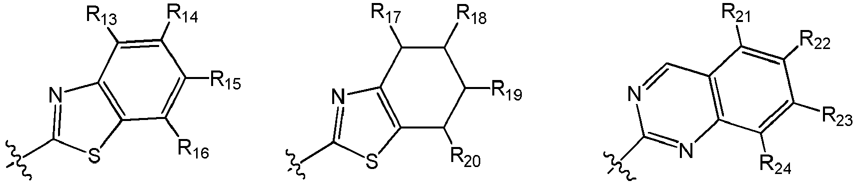 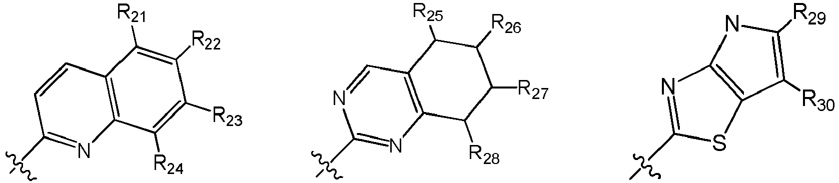 (wherein R₇ to R₃₀ are each independently hydrogen, halogen, C₁-C₃ alkyl or C₁-C₃ alkoxy).

4. The compound, the stereoisomer or the pharmaceutically acceptable salt thereof according to claim 1, wherein A is selected from the group consisting of the following cyclic groups: 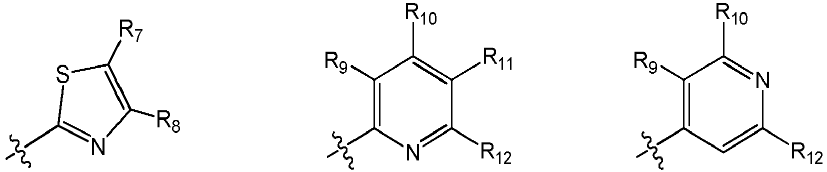 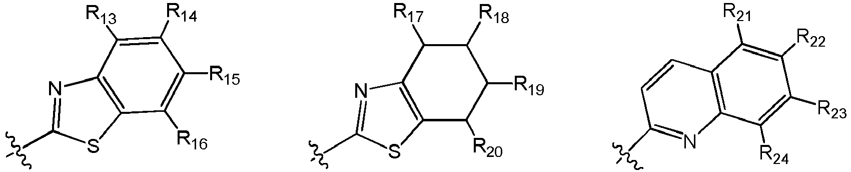 (wherein R₇ to R₂₄ are each independently hydrogen, halogen, C₁-C₃ alkyl or C₁-C₃ alkoxy).

5. The compound, the stereoisomer or the pharmaceutically acceptable salt thereof according to claim 1, wherein A is selected from the group consisting of the following cyclic groups: 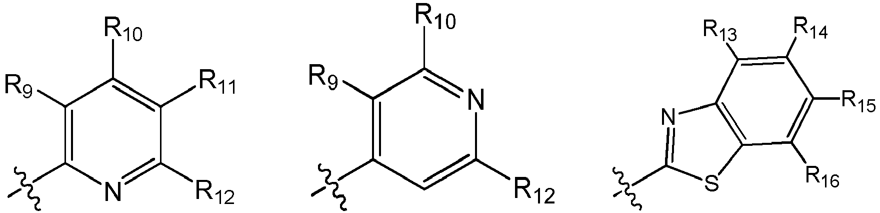 (wherein R₉ to R₁₆ are each independently hydrogen, halogen, C₁-C₃ alkyl or C₁-C₃ alkoxy).

6. The compound, the stereoisomer or the pharmaceutically acceptable salt thereof according to claim 1, wherein R₂ and R₃ are each independently F, Cl or Br.

7. The compound, the stereoisomer or the pharmaceutically acceptable salt thereof according to claim 1, wherein the compound is selected from the group consisting of the following compounds:
N-(5-bromo-6-methylpyridin-2-yl)-2-(1-methyl-1H-indol-3-yl)acetamide,
N-(5-bromo-6-methylpyridin-2-yl)-2-(1H-indol-3-yl)acetamide;
N-(5-bromo-6-methylpyridin-2-yl)-2-(5-chloro-1H-indol-3-yl)acetamide;
N-(benzo[di]thiazol-2-yl)-2-(1H-indol-3-yl)acetamide;
N-(benzo[di]thiazol-2-yl)-2-(5-chloro-1H-indol-3-yl)acetamide;
N-(5-chloro-6-fluoropyridin-2-yl)-2-(1H-indol-3-yl)acetamide;
2-(5-chloro-1H-indol-3-yl)-N-(5-chloro-6-fluoropyridin-2-yl)acetamide;
2-(1H-indol-3-yl)-N-(3,4,5-trimethoxyphenyl)acetamide;
2-(5-chloro-1H-indol-3-yl)-N-(3,4,5-trimethoxyphenyl)acetamide;
N-(3,5-dichlorophenyl)-2-(1H-indol-3-yl)acetamide;
2-(5-chloro-1H-indol-3-yl)-N-(3,5-dichlorophenyl)acetamide;
N-(5-bromo-6-methylpyridin-2-yl)-2-(5-fluoro-1H-indol-3-yl)acetamide;
2-(5-chloro-1H-indol-3-yl)-N-(pyridin-4-yl)acetamide;
N-(benzo[di]thiazol-2-yl)-N-methyl-2-(1-methyl-1H-indol-3-yl)acetamide;
N-(benzo[di]thiazol-2-yl)-2-(1H-indol-3-yl)-N-methyl acetamide;
N-(benzo[di]thiazol-2-yl)-2-(5-chloro-1H-indol-3-yl)-N-methyl acetamide;
N-(5-chloro-6-fluoropyridin-2-yl)-2-(1H-indol-3-y1)-N-methyl acetamide;
2-(5-chloro-1H-indol-3-yl)-N-(5-chloro-6-fluoropyridin-2-yl)-N-methyl acetamide;
N-(benzo[di]thiazol-2-yl)-2-(5-chloro-1-methyl-1H-indol-3-yl)-N-methyl acetamide;
N-(5-chloro-6-fluoropyridin-2-yl)-N-methyl-2-(1-methyl-1H-indol-3-yl)acetamide;
2-(5-chloro-1-methyl-1H-indol-3-yl)-N-(5-chloro-6-fluoropyridin-2-yl)-N-methyl acetamide;
2-(5-chloro-1-methyl-1H-indol-3-yl)-N-(5-chloro-6-fluoropyridin-2-yl)acetamide;
N-(benzo[di]thiazol-2-yl)-2-(1-methyl-1H-indol-3-yl)acetamide;
N-(benzo[di]thiazol-2-yl)-2-(5-fluoro-1H-indol-3-yl)-N-methyl acetamide;
N-(benzo[di]thiazol-2-yl)-2-(5-chloro-1-methyl-1H-indol-3-yl)acetamide;
N-(benzo[di]thiazol-2-yl)-2-(5-fluoro-1H-indol-3-yl)acetamide;
N-(benzo[di]thiazol-2-yl)-2-(6-chloro-1H-indol-3-yl)acetamide;
2-(5-chloro-1H-indol-3-yl)-N-(thiazol-2-yl)acetamide;
2-(5-chloro-1H-indol-3-yl)-N-(quinolin-2-yl)acetamide; and
2-(5-chloro-1H-indol-3-yl)-N-(4,5,6,7-tetrahydrobenzo[di]thiazol-2-yl)acetamide.

8. A pharmaceutical composition, comprising the compound, the stereoisomer or the pharmaceutically acceptable salt thereof according to any one of claims 1 to 7.

9. The pharmaceutical composition according to claim 8, wherein the composition is used for treating or preventing autoimmune diseases.

10. The pharmaceutical composition according to claim 8, wherein the autoimmune disease is any one selected from the group consisting of multiple sclerosis, inflammatory bowel disease, graft-versus-host disease, asthma, atopy, psoriasis, rheumatoid arthritis, systemic lupus erythematous and type 1 diabetes.

11. The pharmaceutical composition according to claim 8, wherein the composition is used for treating or preventing cancer.

12. The pharmaceutical composition according to claim 11, wherein the cancer is selected from the group consisting of melanoma, colon cancer, liver cancer, gliocytoma, ovarian cancer, colon cancer, head and neck cancer, bladder cancer, kidney cell cancer, stomach cancer, breast cancer, metastatic cancer, prostate cancer, gallbladder cancer, pancreatic cancer, blood cancer, skin cancer and lung cancer.

13. A method for treatment of autoimmune diseases, comprising administering the compound, the stereoisomer or the pharmaceutically acceptable salt thereof according to any one of claims 1 to 7 to a subject in need thereof.

14. The method for treatment of autoimmune diseases according to claim 13, wherein the autoimmune diseases are selected from the group consisting of multiple sclerosis, inflammatory bowel disease, graft-versus-host disease, asthma, atopy, psoriasis, rheumatoid arthritis, systemic lupus erythematous and type 1 diabetes.

15. A method for inducing AHR, comprising administering the compound, the stereoisomer or the pharmaceutically acceptable salt thereof according to any one of claims 1to 7 to a subject in need thereof.

16. A method for inhibiting production of IL-6, comprising administering the compound, the stereoisomer or the pharmaceutically acceptable salt thereof according to any one of claims 1 to 7 to a subject in need thereof.

17. A method for treatment of a cancer, comprising administering the compound, the stereoisomer or the pharmaceutically acceptable salt thereof according to any one of claims 1 to 7 to a subject in need thereof.

18. The method for treatment of a cancer according to claim 17, wherein the cancer is selected from the group consisting of melanoma, colon cancer, liver cancer, gliocytoma, ovarian cancer, colon cancer, head and neck cancer, bladder cancer, kidney cell cancer, stomach cancer, breast cancer, metastatic cancer, prostate cancer, gallbladder cancer, pancreatic cancer, blood cancer, skin cancer and lung cancer.
